(19) Europäisches Patentamt / European Patent Office / Office européen des brevets

(11) **EP 4 306 960 A1**

(12) **EUROPEAN PATENT APPLICATION**
published in accordance with Art. 153(4) EPC

(43) Date of publication:
**17.01.2024 Bulletin 2024/03**

(21) Application number: **22766269.9**

(22) Date of filing: **07.03.2022**

(51) International Patent Classification (IPC):
**G01N 33/574** (2006.01)     **G01N 33/92** (2006.01)
**G01N 30/02** (2006.01)

(52) Cooperative Patent Classification (CPC):
**A61K 45/06; A61P 35/00; G01N 30/02;
G01N 30/04; G01N 30/72; G01N 30/88;
G01N 33/574; G01N 33/92**

(86) International application number:
**PCT/CN2022/079542**

(87) International publication number:
**WO 2022/188746 (15.09.2022 Gazette 2022/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.03.2021  CN 202110249219**

(71) Applicant: **Jiang, Chenghong
Fuzhou, Fujian 350007 (CN)**

(72) Inventor: **Jiang, Chenghong
Fuzhou, Fujian 350007 (CN)**

(74) Representative: **Valea AB
Box 1098
405 23 Göteborg (SE)**

(54) **APPLICATION OF COMPOSITION IN PREPARATION OF DRUG FOR DIAGNOSING INFANTILE HEMANGIOMA AND/OR MONITORING PROGRESS AND PROGNOSIS THEREOF, AND KIT AND DRUG**

(57)     An application of a composition in preparation of a drug for diagnosing infantile hemangioma (IH) and/or monitoring progress and prognosis thereof, and a test kit comprising the composition and instructions. The composition comprises at least one of the following four groups of substances: (1) at least one of a total fatty acid or a total saturated fatty acid or a total mono-unsaturated fatty acid or a specific fatty acid; (2) at least one of specific metabolites; (3) at least one of specific proteins; and (4) at least one of specific lipid subclass substances or lipid molecules. The application and the kit can realize early warning/prediction and early diagnosis of the IH, close monitoring of disease progress, objective evaluation of a treatment effect of the drug, and accurate reflection of prognosis. A drug for treating the IH comprises a substance regulating expression selected from the group consisting of LDHB, KRT1, BCHE, CFHR4, CPQ, APOC-3, ARHGDIB, and LAMAS, thereby providing a new effective way for treatment of the IH.

Figure 18

EP 4 306 960 A1

## Description

### TECHNICAL FIELD

The application relates to the use of a composition in the preparation of a drug for diagnosing hemangioma and/or monitoring its progress and prognosis, as well as a kit and a medicament, more specifically, the application relates to a composition for preparing and diagnosing infantile hemangioma and/or the use of drugs to monitor its progress and prognosis, as well as detection kits and drugs for the treatment of infantile hemangioma.

### BACKGROUND TECHNIQUE

Infantile hemangioma, IH is the most common benign tumor in infants and young children, commonly known as "Red Birthmark", the incidence rate is as high as 1-10%. It usually appears a few weeks after birth as a dot-shaped red mass, which rapidly proliferates in the following months and becomes a red mass. If not treated in time, 10%-15% will develop complications, leading to appearance damage, large-scale ulcer infection, respiratory tract compression and obstruction, and even life-threatening.

Therefore, early diagnosis and timely therapeutic intervention are crucial to obtain a satisfactory prognosis. Infantile hemangioma is difficult to distinguish from other common skin diseases such as neonatal erythema (Nevus Simplex, NS) in the early stage of disease. At present, there is no effective prediction/early warning and early differential diagnosis technology for infantile hemangioma. However, due to the rapid development of infantile hemangioma, once the timing of treatment is delayed, serious consequences will result. On the contrary, if other common skin diseases are misdiagnosed as infantile hemangioma, resulting in overtreatment, it may also cause unnecessary iatrogenic damage to children.

In addition, the conventional method, which relies on measuring the size of the hemangioma and observing the texture and color changes by the doctor, is still used to evaluate the treatment effect of drugs against infantile hemangioma. Large measurement errors still exist. Therefore, evaluating the curative effect timely, accurately, and objectively, and correctly judging the end point of the course of the disease are still challenging.

Also, a deep infantile hemangioma may be easily missed or misdiagnosed because there are no obvious symptoms on the body surface or only a bulge on the skin surface. At present, there is a lack of early and accurate differential diagnosis technology. In addition, patients with infantile hemangioma have a high recurrence rate. If surface symptoms disappear or stop developing, the tumor may still recur after withdrawal. How to determine the endpoint of medication is also a challenge that existing technology has not yet overcome.

In summary, there is an urgent need for rapid, accurate, sensitive and quantifiable infantile hemangioma prediction/early warning and early diagnosis technology, technology for monitoring the progress of the disease, and effective prediction technology for the effect of drug treatment and recurrence after stopping treatment.

### SUMMARY OF THE INVENTION

The purpose of the present invention is to overcome the existing international lack of predictive and early warning technologies for infantile hemangiomas, as well as the shortcomings of low detection rate, poor detection sensitivity, easy confusion with other skin diseases with similar appearances, large measurement errors in technology for treatment effectiveness and prognosis evaluation, and the inability to quantify detection indicators, in order to provide a detection technology that is fast, accurate sensitivity and quantitative prediction/warning of infantile hemangioma, precise early diagnosis, close monitoring of disease progression, objective evaluation of drug treatment efficacy, and accurate reflection of prognosis.

The present invention provides use of a composition for manufacturing a medicament for diagnosing infantile hemangioma and/or monitoring its progress and prognosis is characterized in that the composition includes at least one of the following four groups of substances:

(1) Fatty acid, wherein the fatty acid is total fatty acid or total saturated fatty acid or total monounsaturated fatty acid or at least one of the fatty acids selected from the group consisting of C10:0, C11:0, C12:0, C14:0, C15:0, C15:1N5, C16:0, C17:0, C18:0, C18:2N6, C20:0, C20:3N3, C20:5N3, C21:0, C22:1N9, C23:0, C24:0 and C8:0;

(2) Metabolites, wherein the metabolites is at least one of the metabolites selected from the group consisting of (3-carboxypropyl)trimethylammonium cation, $\alpha$-D-(+)-talose, 1-aminocyclopropanecarboxylic acid, 1-meat Myristoyl-sn-glycero-3-phosphocholine, 1-oleoyl-sn-glycero-3-phosphocholine, 1-palmitoyl-sn-glycero-3-phosphocholine, 1-stearoyl-sn -Glycero-3-phosphocholine, bilirubin, choline, D-2-pipercolic acid, DL-indole-3-lactic acid, glycylglutamate, glycerophosphorylcholine, isomaltose, L - Arginine, L-glutamic acid, L-phenylalanine, L-tryptophan, NG,NG-dimethyl-L-arginine, tyramine, phenylacetic acid, (S)-lime Acid, 1-oleoyl-L-alpha-lysophosphatidic acid, 1-palmitoyl lysophosphatidic acid, alpha-mangostin, arachidonic acid (peroxide free), citramalic acid, cyclamate /salt, D-galactate/salt, D-threitol, D(-)-beta-hydroxybutyric acid, inositol galactoside, glyceric acid, L-aspartic acid, L-carnosine, L - In the group consisting of malic acid, N-acetyl-L-aspartic acid, N-acetylneuraminic acid, norethindrone acetate, phosphorylcholine, sn-glycero-3-phosphoethanolamine and succinate/salt;

(3) Protein, wherein the protein is at least one of the proteins selected from the group consisting of COLEC10, CPQ, LDHB, ACAN, KRT1, ARHGDIB, POSTN, LAMAS, CPN1, PROC, C1S, HPX, SUPT6H, BCAR3, BCHE, SOX30, C1R, PON1, CFHR4, PROZ, IGKV1D-16, APOC-3, COMP, SELENOP, PMFBP1, GSN, SELL, FUCA2, HLA-B, FGFBP2, FKBP1A, HSPE1, SAA1, PGD, CDH1, SOD1, ISLR, CORO1A, ZYX, RLBP1, IDH1, IGKV6-21, SORL1, FAM3C, CST6, COTL1, PIGR, A0A1W6IYJ2, PRDX1, CAT, PROS1, CD5L, HMFT1766, C1QA, KRT2, and APP; and

(4)Lipid subclass substances or lipid molecules, wherein the lipid subclass substance is at least one of the lipid subclass substances selected from the group consisting of acetylcarnitine, ceramide, monosaccharide ceramide, disaccharide ceramide, glycosyl ceramide series, trisaccharide Ceramide, cholesteryl ester, cardiolipin, ganglioside, lysophosphatidylcholine, lysophosphatidylethanolamine, monogalactosyldiacylglyceride, phosphatidic acid, phosphatidylcholine, phosphatidylethanolamine, phytosphingosine, phosphatidylinositol, phosphatidylserine, sphingomyelin and triglyceride, and wherein the lipid molecule is at least one of the lipid molecules selected from the group consisting of PS(39:4)-H, SM(d45:6)+H, PC(14:0/20:4)+HCOO, SM(d20:0/18:1)+HCOO, PS(49:4)-H, LPC(26:0)+HCOO, SM(d20:0/16: 0)+HCOO, PA(50:4)-H, LPC(20:4)+HCOO, PC(36:5)+H, LPC(20:1)+HCOO, PC(38:8)+H, PC(46:5 )+H, PE(18:0p/22:5)-H, SM(d22:0/16:0)+HCOO, PC(15:0/20:4)+HCOO, SM(d18:0/20:4)+HCOO, LPC(22:4)+HCOO, PI(18:0/22:4)-H, PC(16:1/18:2)+HCOO, PS(40:4)-H, Cer(d20:0/ 24:1)+H, SM(d37:0)+HCOO, PC(17:1/16:0)+HCOO, SM(d20:0/22:5)+HCOO, PC(44:11)+H, SM(d32:0)+HCOO, LPC(22:1)+H, PC(33:0e)+H, SM(d56:2+pO)+H, SM(d42:1)+HCOO, LPC(17:1)+HCOO, SM(d17:0/18:2)+HCOO, Cer(d18:1/24:0)+H, SM(d36:3)+HCOO, AcCa(14:2)+H, Cer(d18:1/24:0 O)+H, PE(20:0p/20:4)-H, LPC(26:1)+HCOO, PC(37:5)+H, SM(d44:1)+HCOO, Cer(d18:1/26:1)+H, PE(40 :6e)+H, TG(18:1/20:2/22:4)+NH4, SM(d34:3)+H, PE(40:4)-H, SM(d22:0/18:2)+HCOO, Cer(d18:0/22:0)+H, SM(d44:0)+HCOO, PI(16:1/20:4)-H, PC(20:3/20:4)+HCOO, PE(38:1p )-H and SM(d39:0)+HCOO.

The present invention also provides a detection kit, which includes the composition described in the present invention and instructions for using the kit.

The above-mentioned use and detection kit of the present invention can quickly, accurately, sensitively and quantitatively realize the prediction/early warning, early diagnosis, close monitoring of the progress of the disease course, objectively evaluate the therapeutic effect of drugs, and accurately reflect the prognosis of infantile hemangioma .

In addition, the present application also provides a drug for treating infantile hemangioma including substances that regulate the expression of LDHB, KRT1, BCHE, CFHR4, CPQ, APOC-3, ARHGDIB and LAMAS, thereby providing new effective way.

## DESCRIPTION OF DRAWING

Fig. 1 is the lesion photograph at the cubital fossa of the neonate suffering from infantile hemangioma involved in Example 1 of the present invention at the age of 6 weeks;
Fig. 2 is the photograph of the lesion on the back of a newborn with infantile hemangioma involved in Example 2 of the present invention at 6 weeks of birth;
Fig. 3 is the photo of the head lesion of a newborn suffering from neonatal erythema involved in Example 3 of the present invention at 6 weeks after birth;
Fig. 4 is the photo of the head lesion of a newborn suffering from neonatal erythema involved in Example 4 of the present invention at 6 weeks after birth;
Fig. 5 is the photo of the lesion at the cubital fossa of a newborn with infantile hemangioma involved in Example 1 of the present invention at 12 weeks of birth;
Fig.6 is a photo of the back lesion of a newborn with infantile hemangioma involved in Example 2 of the present invention at 12 weeks of birth;
Fig.7 is a photo of the head lesion of a newborn with neonatal erythema involved in Example 3 of the present invention at 12 weeks of birth;
Fig. 8 is a photo of the head lesion of a newborn with neonatal erythema involved in Example 4 of the present invention at 12 weeks of birth;
Fig. 9 is a comparative photo of the lesion at the eyelid of a newborn suffering from an infantile hemangioma related to Example 7 of the present invention before and after treatment and before and after drug withdrawal;
Fig. 10 Statistical results of lipid subclasses (lipid class) and the number of lipid molecules (lipid species) identified in each category;

Fig. 11 control group and IH group total lipid content figure;
Fig. 12 control group and IH group general difference lipid subclass content graph;
Fig. 13 control group and IH group significant difference lipid subclass content figure;
Fig. 14 High-throughput swissport proteomics detection fitting model ROC curve;
Fig. 15 High-throughput uniport proteomics detection fitting model ROC curve;
Fig. 16 metabolite group fitting model ROC curve;
Fig. 17 The ROC curve of the IH group and the control group fitting model in the fatty acid group;
Fig. 18 ROC curve of the fitting model of the IH group and the NS group in the fatty acid group.
Fig. 19 ROC curve of IH group and control group involving 5 fatty acids in the training set fitting model.
Fig. 20 ROC curve of IH group and control group involving 5 fatty acids.

DETAILED WAYS

The invention provides use of a composition for manufacturing a medicament for diagnosing infantile hemangioma and/or monitoring its progress and prognosis is characterized in that the composition includes at least one of the following four groups of substances:

(1) Fatty acid, wherein the fatty acid is total fatty acid or total saturated fatty acid or total monounsaturated fatty acid or at least one of the fatty acids selected from the group consisting of C10:0, C11:0, C12:0, C14:0, C15:0, C15:1N5, C16:0, C17:0, C18:0, C18:2N6, C20:0, C20:3N3, C20:5N3, C21:0, C22:1N9, C23:0, C24:0 and C8:0;
(2) Metabolites, wherein the metabolites is at least one of the metabolites selected from the group consisting of (3-carboxypropyl)trimethylammonium cation, $\alpha$-D-(+)-talose, 1-aminocyclopropanecarboxylic acid, 1-meat Myristoyl-sn-glycero-3-phosphocholine, 1-oleoyl-sn-glycero-3-phosphocholine, 1-palmitoyl-sn-glycero-3-phosphocholine, 1-stearoyl-sn -Glycero-3-phosphocholine, bilirubin, choline, D-2-pipercolic acid, DL-indole-3-lactic acid, glycylglutamate, glycerophosphorylcholine, isomaltose, L - Arginine, L-glutamic acid, L-phenylalanine, L-tryptophan, NG,NG-dimethyl-L-arginine, tyramine, phenylacetic acid, (S)-lime Acid, 1-oleoyl-L-alpha-lysophosphatidic acid, 1-palmitoyl lysophosphatidic acid, alpha-mangostin, arachidonic acid (peroxide free), citramalic acid, cyclamate /salt, D-galactate/salt, D-threitol, D(-)-beta-hydroxybutyric acid, inositol galactoside, glyceric acid, L-aspartic acid, L-carnosine, L - In the group consisting of malic acid, N-acetyl-L-aspartic acid, N-acetylneuraminic acid, norethindrone acetate, phosphorylcholine, sn-glycero-3-phosphoethanolamine and succinate/salt;
(3) Protein, wherein the protein is at least one of the proteins selected from the group consisting of COLEC10, CPQ, LDHB, ACAN, KRT1, ARHGDIB, POSTN, LAMAS, CPN1, PROC, C1S, HPX, SUPT6H, BCAR3, BCHE, SOX30, C1R, PON1, CFHR4, PROZ, IGKV1D-16, APOC-3, COMP, SELENOP, PMFBP1, GSN, SELL, FUCA2, HLA-B, FGFBP2, FKBP1A, HSPE1, SAA1, PGD, CDH1, SOD1, ISLR, CORO1A, ZYX, RLBP1, IDH1, IGKV6-21, SORL1, FAM3C, CST6, COTL1, PIGR, AOA1W6IYJ2, PRDX1, CAT, PROS1, CD5L, HMFT1766, C1QA, KRT2, and APP; and
(4)Lipid subclass substances or lipid molecules, wherein the lipid subclass substance is at least one of the lipid subclass substances selected from the group consisting of acetylcarnitine, ceramide, monosaccharide ceramide, disaccharide ceramide, glycosyl ceramide series, trisaccharide Ceramide, cholesteryl ester, cardiolipin, ganglioside, lysophosphatidylcholine, lysophosphatidylethanolamine, monogalactosyldiacylglyceride, phosphatidic acid, phosphatidylcholine, phosphatidylethanolamine, phytosphingosine, phosphatidylinositol, phosphatidylserine, sphingomyelin and triglyceride, and wherein the lipid molecule is at least one of the lipid molecules selected from the group consisting of PS(39:4)-H, SM(d45:6)+H, PC(14:0/20:4)+HCOO, SM(d20:0/18:1)+HCOO, PS(49:4)-H, LPC(26:0)+HCOO, SM(d20:0/16: 0)+HCOO, PA(50:4)-H, LPC(20:4)+HCOO, PC(36:5)+H, LPC(20:1)+HCOO, PC(38:8)+H, PC(46:5 )+H, PE(18:0p/22:5)-H, SM(d22:0/16:0)+HCOO, PC(15:0/20:4)+HCOO, SM(d18:0/20:4)+HCOO, LPC(22:4)+HCOO, PI(18:0/22:4)-H, PC(16:1/18:2)+HCOO, PS(40:4)-H, Cer(d20:0/24:1)+H, SM(d37:0)+HCOO, PC(17:1/16:0)+HCOO, SM(d20:0/22:5)+HCOO, PC(44:11)+H, SM(d32:0)+HCOO, LPC(22:1)+H, PC(33:0e)+H, SM(d56:2+pO)+H, SM(d42:1)+HCOO, LPC(17:1)+HCOO, SM(d17:0/18:2)+HCOO, Cer(d18:1/24:0)+H, SM(d36:3)+HCOO, AcCa(14:2)+H, Cer(d18:1/24:0 O)+H, PE(20:0p/20:4)-H, LPC(26:1)+HCOO, PC(37:5)+H, SM(d44:1)+HCOO, Cer(d18:1/26:1)+H, PE(40 :6e)+H, TG(18:1/20:2/22:4)+NH4, SM(d34:3)+H, PE(40:4)-H, SM(d22:0/18:2)+HCOO, Cer(d18:0/22:0)+H, SM(d44:0)+HCOO, PI(16:1/20:4)-H, PC(20:3/20:4)+HCOO, PE(38:1p )-H and SM(d39:0)+HCOO.

In the present invention, the C10:0 refers to decanoic acid. The C11:0 refers to undecanoic acid. The C12:0 refers to dodecanoic acid. The C14:0 refers to myristic acid. The C15:0 refers to pentadecanoic acid . The C15:1N5 refers to cis-10-pentadecene acid. The C16:0 refers to palmitic acid. The C17:0 refers to heptadecanoic acid. The C18:0 refers to stearic acid. The C18:2N6 refers to linoleic acid. The C20:0 refers to arachidic acid. The C20:3N3 refers to cis-11,14,17-eicosatrienoic acid. The C20:5N3 refers to cis-5,8,11,14,17-eicosapentaenoic acid. The C21:0 refers to heneicosanic acid . The C22:1N9 refers to erucic acid. The 23:0 refers to tricosanic acid. The C24:0 refers to tetracosanoic acid. The C8:0 refers to octanoic acid. The fatty acid of the invention is preferably selected from at least one of the group consisting of C8:0, C10:0, C11:0, C16:0, C20:5N3, C22:1N9, C23:0 and C24:0. It is further preferably selected from at least one of the group consisting of C8:0, C10-0-C16:0, C20:0-C20: SN3, C22:1N9, C23:0, C24:0; It is preferably selected from at least one of the group consisting of C20:5N3, C22:1N9, C23:0 and C24-0. Preferably, the fatty acid of the invention is selected from the group consisting of C10:0, C14:0, C16:0, C18:0, C18:2N6.C20:0, C20:1N9, C20:5N3-C22:1N9, C24:0, and C8:0.The more preferable fatty acid of the invention is selected from the group consisting of C10:0, C16:0, C20:0, C20:5N3 and C22:1N9.

Preferably, the metabolite is at least one selected from the group consisting of choline, 1-aminocyclopropanecarboxylic acid, 1-myristoyl-sn-glycero-3-phosphocholine , D-2-pipecolinic acid, DL-indole-3-lactic acid, glycyl glutamic acid (Gly-Glu), L-Glutamate, L-Phenylalanine, L-Tryptophan, Tyramine, Phenylacetic acid, Glyceric acid, Cyclohexylsulfamate/salt, D-galactate/salt and sn-glycerol-3-phosphoethanolamine. The most preferable metabolites are at least one selected from the group consisting of 1-myristoyl-sn-glycero-3-phosphocholine, glycyl glutamic acid (Gly-Glu), L-phenylalanine, DL-Indole-3-lactic acid, L-Tryptophan, Phenylacetic acid, and Glyceric acid.

Among the proteins involved in the present invention, the COLEC10 refers to collagen lectin-10 (Collectin-10). The CPQ refers to carboxypeptidase Q . The LDHB refers to L-lactate dehydrogenase B chain . The ACAN refers to proteoglycan core protein. The KRT1 refers to keratin, type II cytoskeletal 1 . The POSTN refers to periostin. The LAMA5 refers to laminin subunit alpha-5. The CPN1 refers to carboxypeptidase Ncatalytic chain. The PROC refers to vitamin K-dependent protein C. The C1S refers to the complement C1s subcomponent . The HPX refers to hemopexin. The SUPT6H refers to transcription elongation factor SPT6. The BCAR3 refers to breast cancer anti-estrogen resistance protein 3. The BCHE refers to Cholinesterase. The SOX30 refers to the transcription factor SOX-30. The C1R refers to a complement C1r subcomponent. The PON1 refers to serum paraoxonase/arylesterase 1. The CFHR4 refers to complement factor H-related protein 4. The PROZ refers to vitamin K-dependent protein Z. The IGKV1D-16 refers to the immunoglobulin kappa variable 1D-16. The APOC-3 refers to apolipoprotein C-III. The COMP refers to cartilage oligomeric matrix protein. The SELENOP refers to selenoprotein P. The PMFBP1 refers to polyamine-modulated factor 1-binding protein 1. The GSN refers to gelsolin . The SELL refers to L-selectin. The FUCA2 refers to plasma α-L-fucosidase. The HLA-B refers to human leukocyte antigen B antigen. The FGFBP2 refers to fibroblast growth factor-binding protein 2. The FKBP1A refers to peptidyl-prolyl cis-trans isomerase. The HSPE1 refers to heat shock protein 10 . The SAA1 refers to serum amyloid A-1. The PGD refers to 6-phosphogluconate dehydrogenase . The CDH1 refers to Cadherin-1. The SOD1 refers to superoxide dismutase [Cu-Zn]. The ISLR refers to the immunoglobulin superfamily containing leucine-rich rich in leucine repeat sequence. The COROIA refers to Coronin-1A. The ZYX refers to Zexin. The RLBP1 refers to retinal binding protein 1. The IDH1 refers to cytoplasmic isocitrate dehydrogenase. The IGKV6-21 refers to immunoglobulin kappa variable region 6-21. The SORL1 refers to a sortin-related receptor . The FAM3C refers to FAM3C protein. The CST6 refers to cysteine protease inhibitor-M . The COTL1 refers to actin-like protein. The PIGR refers to polymeric immunoglobulin receptor. The AOA1W6IYJ2 refers to the N90-VRC38.05 heavy chain variable region. The PRDX1 refers to peroxide reductase-1. The CAT refers to catalase. The PROS1 refers to vitamin K-dependent protein S . The CD5L refers to CD5 antigen-like. The HMFT1766 refers to leucine-rich alpha-2-glycoprotein . The C1QA refers to the complement C1q-α subcomponent . The KRT2 refers to keratin 2. The APP refers to amyloid-beta precursor protein.

Wherein LDHB, KRT1, BCHE, CFHR4 and CPQ are up-regulated proteins, while APOC-3, ARHGDIB and LAMA5 are down-regulated proteins. The protein is further preferably at least one selected from the group consisting of HSPE1, PGD, CDH1, ISLR, CPQ, IDH1, PIGR, POSTN, PROC, PROZ, PMFBP1, SELL, A0A1W6IYJ2, CAT, PROS1, CD5L, C1QA, KRT1 and FGFBP2. The protein is most preferably at least one selected from the group consisting of HSPE1, CDH1, ISLR, IDH1, COTL1 and FGFBP2.

The lipids (Lipids) described in the present invention are a class of hydrophobic or amphiphilic small molecules, including eight categories (named by LIPID MAPS ® system) (Fahy et al., 2009): fatty acids (such as linoleic acid, arachidic acid, etc. ), glycerolipids (such as TG, DG, etc.), glycerophospholipids (such as PC, PE, PG, PA, etc.), sphingolipids (such as Cer, SM, etc.), sterol lipids (such as sterol esters, etc.) , glycolipids (such as MGDG, SQDG, etc.), pregnenolone lipids (such as CoA, etc.) and polyvinyls (antibiotics, etc.). Lipids are the main components of biological membrane structures (such as outer membranes, mitochondria, exosomes, endoplasmic reticulum, exosomes and other subcells), as well as small signaling molecules and energy substances. Specifically, the names of lipid subclasses involved in the present invention and their English abbreviations are shown in Table 1 below:

Table 1 The names and English abbreviations of lipid subclasses

| category | subclass | subclass abbreviation | Molecular species | Summary of molecular species |
|---|---|---|---|---|
| | Acetylcarnitine | AcCa | 13 | 13 |

(continued)

| category | subclass | subclass abbreviation | Molecular species | Summary of molecular species |
|---|---|---|---|---|
| | Wax esters | WE | 2 | 2 |
| Glycerophospholi pid GP | Cardiolipin | CL | 1 | 379 |
| | Lysophosphatidylcholi ne | LPC | 45 | |
| | Lysophosphatidylethan olamine | LPE | 16 | |
| | Lysophosphatidylinosit ol | LPI | 2 | |
| | Phosphatidic acid | Well | 1 | |
| | Phosphatidylcholine | PC | 208 | |
| | Phosphatidylethanolam ine | ON | 63 | |
| | Phosphatidylinositol | PI | 22 | |
| | Phosphatidylserine | PS | 21 | |
| Glycerides GL | diglycerides | DG | 18 | 187 |
| | Triglycerides | TG | 169 | |
| Sterol Lipid ST | cholesteryl ester | That | 7 | 7 |
| Sphingolipid SP | Ceramide | Cer | 59 | 211 |
| | Gangliosides | GM3 | 11 | |
| | Phytosphingosine | phSM | 8 | |
| | Sphingomyelin | SM | 130 | |
| | Sphingosine | So | 3 | |
| Glycolipids | monosaccharide ceramide | CerG1 | 11 | 21 |
| | Bisaccharylceramide | CerG2 | 4 | |
| | Glycosylceramide series | CerG2GNAcl | 2 | |
| | Triosylceramide | CerG3 | 2 | |
| | monogalactose diacylglyceride | MGDG | 2 | |
| Pregnenolone Lipid PR | coenzyme | Co | 1 | 1 |

The lipid subclass substance of the present invention is further preferably selected from the group consisting of Cer, ChE, LPC, LPE, PA, PC, PE, PI, PS, and SM. Most preferably it is selected from the group consisting of LPC, LPE, PA, PC and PE. Preferably, the lipid molecule is selected from the group of consisting of PS(39:4)-H, SM(d45:6)+H, PC(14:0/20:4)+HCOO, SM(d20:0/18:1)+HCOO, PS(49:4)-H, LPC(26:0)+HCOO, SM(d20:0/16: 0)+HCOO, PA(50:4)-H, LPC(20:4)+HCOO, PC(36:5)+H, LPC(20:1)+HCOO, PC(38:8)+H, PC(46:5 )+H, PE(18:0p/22:5)-H, SM(d22:0/16:0)+HCOO, PC(15:0/20:4)+HCOO, SM(d18:0/20:4)+HCOO, LPC(22:4)+HCOO, PI(18:0/22:4)-H, PC(16:1/18:2)+HCOO, PS(40:4)-H, Cer(d20:0/ 24:1)+H, SM(d37:0)+HCOO, PC(17:1/16:0)+HCOO, SM(d20:0/22:5)+HCOO, PC(44:11)+H, SM(d32:0)+HCOO, LPC(22:1)+H, PC(33:0e)+H, SM(d56:2+pO)+H, SM(d42:1)+HCOO, LPC(17:1)+HCOO, SM(d17:0/18:2)+HCOO, Cer(d18:1/24:0)+H, SM(d36:3)+HCOO, Ac-Ca(14:2)+H, Cer(d18:1/24:0 O)+H, PE(20:0p/20:4)-H, LPC(26:1)+HCOO, PC(37:5)+H, SM(d44:1)+HCOO, Cer(d18:1/26:1)+H, PE(40 :6e)+H, TG(18:1/20:2/22:4)+NH4, SM(d34:3)+H, PE(40:4)-H, SM(d22:0/18:2)+HCOO, Cer(d18:0/22:0)+H, SM(d44:0)+HCOO, PI(16:1/20:4)-H, PC(20:3/20:4)+HCOO, PE(38:1p )-H and SM(d39:0)+HCOO. Further preferably, the lipid molecule is selected from: PS(39:4)-H, SM(d45:6)+H, PC(14:0/20:4)+HCOO, SM(d20:0/18:1)+HCOO, PS(49:4)-H, LPC(26:0)+HCOO, SM(d20:0/16 :0)+HCOO, PA(50:4)-H and LPC(20:4)+HCOO. Most preferably, the lipid molecule selected from PS(39:4)-H, SM(d45:6)+H, PC(14:0/20:4)+HCOO and SM(d20:0/18:1)+HCOO.

The inventor of the present invention collects umbilical cord blood of 3089 cases of newborns,including 63 cases of positive samples of infantile hemangioma. Thousands of metabolites in healthy newborns, neonatal erythema patients, and infant hemangioma patients were analyzed and compared using high-throughput metabolomics, fatty acid profile targeting quantitative metabolomics, general metabolomics, proteomics, and lipidomics. Specific fatty acids, metabolites, proteins and lipid subclass substances or lipid molecules listed above have been found to be useful in early warning/prediction, early diagnosis and/or monitoring of the progress and prognosis of infantile-age hemangioma, generating startling specificity and sensitivity.

Any of the fatty acids, metabolites, proteins and lipid subclasses or lipid molecules listed here in this application can be used alone to determine whether infantile hemangioma is present and whether the drug for treating infantile hemangioma is curative. In order to further verify the test results, two or more substances can be used for mutual confirmation. Preferably, the fatty acid is at least 2 of the fatty acids defined in (1); the metabolite is at least 2 of the metabolites defined in (2); the protein is the at least 2 of the proteins defined in (3); or the lipid subtypes or lipid molecules are at least 2 of the lipid subtypes or lipid molecules defined in (4). Further preferably, the fatty acid is at least 3 of the fatty acids defined in (1); the metabolite is at least 3 of the metabolites defined in (2); the protein is the At least 3 of the proteins defined in (3); or the lipid subtypes or lipid molecules are at least 3 of the lipid subtypes or lipid molecules defined in (4). Considering the cost and detection efficiency, the fatty acid is 1 to 6 of the fatty acids defined in (1); the metabolite is 1 to 6 of the metabolites defined in (2). 6 types; or the protein is 1 to 6 of the proteins defined in (3); Or the lipid subclass substance or lipid molecule is one to six of the lipid subclass substance or lipid molecule defined in said (4).

Preferably, the fatty acid in (1) is selected from the group consisting of C8:0, C10:0, C11:0, C16:0, C20:0, C20:5N3, C22:1N9, C23:0 and C24:0; the metabolites in (2) are selected from the group consisting of choline, 1-aminocyclopropanecarboxylic acid, 1-myristoyl-sn-glycero-3-phosphocholine, D-2-pipericolic acid, DL-indole-3-lactic acid, glycyl glutamic acid, L-glutamic acid, L-phenylalanine, L-tryptophan, tyramine, glyceric acid, cyclamate, D - galactate/salt and sn-glycero-3-phosphoethanolamine; said (3) protein is selected from the group consisting of HSPE1, PGD, CDH1, ISLR, CPQ, IDH1, PIGR, POSTN, PROC, PROZ, PMFBP1, SELL, A0A1W6IYJ2, CAT, PROS1, CD5L, C1QA, KRT1 and FGFBP2; or the (4) Lipid subclass substances selected from the group consisting of Cer, ChE, LPC, LPE, PA, PC, PE, PI, PS and SM, the lipid molecule is selected from PS(39:4)-H, SM(d45:6)+H, PC(14:0/20:4) +HCOO, SM (d20:0/18:1) +HCOO, PS(49:4)-H, LPC(26:0) +HCOO, SM(d20:0/16:0) +HCOO, PA(50:4)-H and LPC (20:4) +HCOO. Further preferably, the fatty acid in (1) is selected from the group consisting of C20:5N3, C22:1N9, C23:0 and C24:0; The metabolites in said (2) are selected from the group consisting of 1-myridamyl-Sn-glycero-3 -phosphate choline, glycine glutamic acid, L-phenylalanine, DL-hondole-3-lactic acid, L-tryptophan and glycolic acid. The (3) protein is selected from the group consisting of HSPE1, CDH1, ISLR, IDH1, COTL1 and FGFBP2; or the (4) lipid subclass is selected from the group consisting of LPC, LPE, PA, PC and PE or said lipid molecules are selected from PS(39:4)-H, SM(d45:6)+H, PC(14:0/20:4)+HCOO and SM(d20:0/18:1)+HCOO .

The present application also provides a detection kit, which includes the composition of the present invention and instructions for using the kit. Fatty acids, metabolites and lipid subclasses or lipid molecules of the present application can be determined using conventional methods, for example, spectrophotometry, gas chromatography, liquid chromatography, gas-liquid chromatography, mass spectrometry, gas chromatography Combined use, liquid chromatography-mass spectrometry routine serum total lipid determination, etc. Similarly, the protein can also be determined by conventional methods. Kits may optionally include other reagents, such as buffers, salts, enzymes, enzyme cofactors, substrates, detection reagents, etc., to perform a diagnostic assay or to facilitate quality control assessments. Other components such as buffers and solutions (eg, pretreatment reagents) for isolating and/or processing test samples may also be included in the kit. Optionally, the kit may also contain at least one calibrator or control. Any calibrators or controls can be included in the kit. The kit may additionally include one or more other controls. One or more of the kit components may be lyophilized, and the kit may further include reagents suitable for reconstituting the lyophilized components.

The various components of the kit are optionally provided in suitable containers. One or more of the containers may be a microtiter plate. Kits may further include containers for holding or storing samples (eg, containers or cartridges for blood or urine samples). Kits may also optionally include reaction vessels, mixing vessels, and other components to facilitate reagent or test sample preparation, as appropriate. Kits can also include one or more instruments, such as syringes, pipettes, forceps, measured spoons, etc., to assist in obtaining test samples.

The instructions for using the kit of the present invention can be provided in paper form or computer-readable form such as disk, CD, DVD, flash memory, online download link, mobile phone APP application and the like. The instructions may include a baseline value data set of normal indicators for comparison.

The samples detected by the kit are selected from the group consisting of whole blood, plasma, serum, lymphocyte suspension, cerebrospinal fluid, bone marrow, pleural effusion, urine, saliva and peritoneal effusion. Samples that do not interfere with the subject are preferred. More preferably, the samples detected by the kit are selected from the group consisting of umbilical cord blood and peripheral blood.

Preferably, the kit is a dry blood patch kit. The dry blood spot is obtained by the dry blood paper method. The principle is that the whole blood drops are added to the filter paper, and the dry blood spot is obtained after drying. After solvent extraction, the information of the components to be detected in the dry blood spot is analyzed by fluorescence reaction method and tandem mass spectrometry to reflect the patient's diagnostic results, such as metabolite content, etc. From the perspective of sample preservation, dry blood stains are more stable than whole blood, and the storage and transportation cost is much lower than whole blood. In addition, dried blood paper can collect a small number of samples, so dried blood paper has specific advantages in the detection that requires multiple samples for diagnosis, especially for neonatal disease screening.

The present application also provides a medicament for treating infantile hemangioma, the medicament includes a substance that regulates the expression of LDHB, KRT1, BCHE, CFHR4, CPQ, APOC-3, ARHGDIB and LAMAS. The inventors of the present invention are the first to discover that the relevant protein is related to infantile hemangioma, and the treatment of infantile hemangioma can be achieved by regulating the expression of the relevant protein.

### EXAMPLE

The present invention will be further described through the following embodiments in conjunction with the accompanying drawings, but the present invention is not limited to the following embodiments.

### EXAMPLE OF SAMPLE COLLECTION

The inventor of the present application collected and preserved 3,089 neonatal umbilical cord blood samples, compared multiple factors and dimensions between patients with the disease and normal newborns, and finally found that there are significant statistical differences in indicators among (1) fatty acids, (2) metabolites, (3) protein and (4) lipid subclasses or lipid molecular.

### (1) Subject recruitment

Make a brochure for the popularization of infantile hemangioma, provide it to the delivery room of the cooperative hospital, distribute the brochure to the expectant mothers and their families, introduce the incidence and treatment of infantile hemangioma to the mothers and their families, and inform the rights and benefits of participating researchers; The purpose of this study and the follow-up time were explained to all patients, ensuring that patients know the importance of adhering to designated follow-up times for early intervention in IH. With the full knowledge of the puerpera and her family members, fully respect her will, whether she agrees or not, will not affect any of her treatment and rights. Under the condition that the parturients and their family members agree to participate in the research, they will sign relevant informed consent forms for the collection of cord blood, placental tissue, maternal and neonatal medical history, and data.

### (2) Collect specimens and record maternal and neonatal information

After the fetus and placenta were delivered and the umbilical cord was severed, and the health of the fetus and the mother was ensured, 30 mL of the discarded umbilical vein and umbilical cord blood in the placenta were collected aseptically, and injected into a coagulation blood collection tube (purchased from Fuzhou Chang Gung Medical Instrument Co., Ltd. (CHANGGENG) ), containing diatomaceous earth), shake slowly. After standing at room temperature for half an hour, the neonatal cord blood was centrifuged at 3000g/min for 15 minutes, and the supernatant was taken and stored at -80°C. A total of 3089 neonatal umbilical cord blood samples were collected.

The clinical diagnosis and treatment data will be established under the guidance of statistical professionals for data entry; the specimens and personal information will be marked with research numbers rather than names.

### (3) Follow-up

Telephone follow-up was conducted at the 6th and 12th week after birth to track the occurrence of IH. The telephone follow-up personnel consisted of two postgraduates. After strict training, the follow-up time avoided the rest time of the newborn's family members. During the follow-up, no newborn was missed. The reason for the newborn who quits midway will be registered. During the follow-up, if the symptom description is consistent with infantile hemangioma, the follow-up staff will ask the parents of the infant to send pictures of the lesion to help the diagnosis, and notify all children who are initially diagnosed as infantile hemangioma to the outpatient clinic for diagnosis.

### (4) Match grouping and control confounding factors

After the follow-up, all the cord blood samples of infantile hemangioma diagnosed through outpatient service were selected as the case group. Then, according to the baby's sex, birth weight, gestational weeks and other relevant information as matching conditions, children without infantile hemangioma were matched as the control group.

**(5) Obtaining sample information**

A total of 63 cases with positive IH samples were collected during follow-up, and 28 cases were randomly selected as the IH group. And according to the infant gender, birth weight, gestational weeks and other relevant information of the IH group, the matching conditions were used as matching conditions, and 32 samples of the control group with the same conditions were screened out. In addition, cord blood from 37 children with neonatal erythema (Nevus Simplex, NS) was collected (NS group), a total of 97 samples. Each umbilical cord blood specimen of the IH group and the control group was divided into 100 μL/tube in a 1.5ml centrifuge tube, and stored at -80°C until testing.

**Table 2**

| Sample grouping | Sample group name | quantity |
| --- | --- | --- |
| 1 | IH group | 28 |
| 2 | control group | 32 |
| 3 | NS group | 37 |

**(6) Introduction to statistical methods and indicators used in the present invention**

Table 3 below shows the results of a specific disease diagnosis using a specific diagnostic method.

**table 3**

| diagnostic test | | gold standard | | total |
| --- | --- | --- | --- | --- |
| | | patient | normal person | |
| Positive | a(true negative) | | b(false positive) | a+ b |
| Negative | | c (false negative) | d (true negative) | c+ d |
| total | | a +c | b +d | a+ b+ c +d |

**Sspecificity:** Sp=d/(bd) $\times$ 100% indicates the ability of the diagnostic test to identify non-patients (that is, the probability that a person who is actually not sick is diagnosed as negative), that is, the true negative rate. The larger the value, the higher the diagnostic test is. (method) is more precise.

**Sensitivity:** Se=a/(ac) $\times$ 100% indicates the ability to diagnose patients, that is, the true positive rate. The larger the value, the more sensitive the diagnostic method is.

**Receiver operating characteristic curve:** ROC curve also known as the sensitivity curve: a coordinate diagram composed of the false positive rate (1-specificity) as the horizontal axis and the true positive rate (sensitivity) as the vertical axis, and the subjects in a specific Curves drawn for different results obtained by using different judgment criteria under stimulus conditions. The horizontal and vertical coordinates can be calculated by software (SPSS\Origin\Graphpad Prism), and the area under the ROC curve can be obtained from the results obtained in the software to compare the diagnostic value of the diagnostic test.

The role of the ROC curve:

1) Find the "best" diagnostic cutoff for a diagnostic test.

2) The area under the ROC curve (AUC) was calculated to evaluate the discriminative power of a diagnostic test.

3) Compare the area under the ROC curve of multiple tests to screen out the best diagnostic scheme.

**Area Under the Curve (AUC):**

The ROC curve AUC can reflect the value of the diagnostic test, usually in the range of 0.5-1. In the case of AUC>0.5, the closer the AUC is to 1, the better the diagnostic effect. 0.5 for a completely worthless diagnosis and 1 for a completely ideal diagnosis.

It is generally believed that: AUC:

Between 0.5-0.7: indicates low diagnostic value and low accuracy;
Between 0.7-0.9: it means that the diagnostic value is medium and has a certain accuracy;
>0.9: indicates high diagnostic value and high accuracy.

**The larger the area under the curve (AUC), the higher the overall diagnostic rate:**

**Significant:** Statistical significance, also known as statistical significance, refers to the risk level to be borne if the null hypothesis is true and we rejected it, also known as probability level, or significance level. The ability to distinguish groups from one another. In statistical hypothesis testing, the recognized probability value of a small probability event is called the significance level of statistical hypothesis testing. The same quantity is measured several times, and then the average value is calculated.

**Examples of fatty acid group indicators:**

**(1) Experimental method**

1. Preparation of standard products

Forty kinds of fatty acid methyl ester mixed standard solution were used, each of which produced ten standard concentration gradients of 0.5mg/L, 1mg/L, 5mg/L, 10mg/L, 25mg/L, 50mg/L, 100mg/L, 250mg/L, 500mg/L and 1000mg/L.
Take 500 $\mu$L of 40 fatty acid methyl ester mixed standards at the same concentration, add 25 $\mu$L of 500 ppm methyl n-nonadecanoate as an internal standard, mix well and add to the injection bottle, enter the GC-MS detection, the injection volume is 1 $\mu$L, split ratio 10:1, split injection.

2. Extraction of metabolites from samples to be tested

The sample was thawed on ice, and 60 $\mu$l of the sample was taken in a 2 mL glass centrifuge tube. Add 1 mL of chloroform-methanol solution, sonicate for 30 min, take the supernatant, add 2 mL of 1% sulfuric acid-methanol solution, place it on a water bath at 80 °C, methylate for half an hour, add 1 mL of n-hexane for extraction, wash with 5 mL pure water, absorb 500 $\mu$L of the supernatant, add 25 $\mu$L of methyl salicylate as an internal standard, mix well and add to the injection bottle, and enter the GC-MS detection. The injection volume is 1 $\mu$L, the split ratio is 10:1, and split injection.

3. Chromatography-mass spectrometry analysis

(1) Gas chromatography conditions: The samples were separated by Agilent DB-WAX capillary column (30 m$\times$0.25 mm ID$\times$0.25 $\mu$m) gas chromatography system. Temperature program: initial temperature 50°C; hold for 3 minutes, then raise the temperature to 220°C at 10°C/min; and maintain for 5 minutes. The carrier gas was helium at a flow rate of 1.0 mL/min. A quality control (QC) sample was set for every 7 experimental samples in the sample queue to detect and evaluate the stability and repeatability of the system.
(2) Mass spectrometry: Agilent 7890/5975C gas-mass spectrometer was used for mass spectrometry. The inlet temperature was 280°C; the ion source temperature was 230°C; the transfer line temperature was 250°C. Electron impact ionization (EI) source, SIM scan mode, electron energy 70eV.
(3) Data processing: MSD ChemStation software was used to extract the chromatographic peak area and retention time. Draw a calibration curve and calculate the content of medium and long-chain fatty acids in the sample.
(4) Statistics: Data analysis was carried out using SPSS software (version 25.0; IBM, Armonk NY, USA). Analysis of variance (ANOVA) was used to analyze the differences among the three groups, and the SNK-q test was further used for multiple comparisons. ROC (receiver operating characteristic curve) analysis was used to judge the diagnostic value of difference variables. Binary logistic regression analysis is used to build a logistic regression model to further improve the predictive sensitivity and specificity, and improve the diagnostic value

**(3) Results**

(1) Test results

A total of 39 medium- and long-chain fatty acids were detected this time, and the sum of fatty acid amounts of five medium- and long-chain fatty acid subclasses was calculated: total_SFA (saturated fatty acid), total_MUFA (monounsaturated fatty acid), total PUFA ( polyunsaturated fatty acids), total N3 (omega-3 cluster fatty acids, the first unsaturated bond in unsaturated fatty acids appears at the third position of the methyl end of the carbon chain) and total N6 (omega-6 cluster fatty acids, in unsaturated fatty acids The first unsaturated bond occurs at the sixth position of the methyl end of the carbon chain).

(2) Analysis of variance: ANOVA was used to compare whether there were differences among the three groups of medium and long-chain fatty acids, and the SNK-q test was further used to make pairwise comparisons among the three groups. The results of variance analysis showed that there were 18 medium and long-chain fatty acids among the 39 medium- and long-chain fatty acids with significant differences among the three groups (P<0.05), namely: C10:0, C11:0, C12:0, and C14:0 , C15:0, C15:1N5, C16:0, C17:0, C18:0, C18:2N6, C20:0, C20:3N3, C20:5N3, C21:0, C22:1N9, C23:0, C24 :0 and C8:0. Moreover, there were also significant differences (P<0.05) in total saturated fatty acids (SFA) and total monounsaturated fatty acids (MUFA) among the five medium and long-chain fatty acid subcategories (see Table 4, unit: $\mu$g/ml).

**Table 4**

| fatty acid | average value (THEM) | median (THEM) | average value (NS) | median (NS) | average value (comparison) | median (comparison) | F | P value |
|---|---|---|---|---|---|---|---|---|
| C10:0 | 0.033 | 0.026 | 0.019 | 0.018 | 0.016 | 0.014 | 6.876 | 0.002 |
| C11:0 | 0.020 | 0.018 | 0.013 | 0.012 | 0.010 | 0.011 | 8.735 | < 0.001 |
| C12:0 | 0.355 | 0.201 | 0.169 | 0.170 | 0.203 | 0.160 | 4.351 | 0.016 |
| C13:0 | 0.272 | 0.100 | 0.107 | 0.106 | 0.107 | 0.098 | 2.598 | 0.080 |
| C14:0 | 3.677 | 3.265 | 3.111 | 2.874 | 2.616 | 2.677 | 4.551 | 0.013 |
| C14:1N5 | 1.947 | 1.226 | 1.283 | 0.971 | 1.299 | 1.215 | 2.539 | 0.085 |
| C15:0 | 0.736 | 0.659 | 0.668 | 0.660 | 0.534 | 0.543 | 4.633 | 0.012 |
| C15:1N5 | 32.961 | 29.701 | 23.354 | 23.955 | 23.096 | 24.300 | 7.170 | 0.001 |
| C16:0 | 107.953 | 109.688 | 96.440 | 92.488 | 84.327 | 89.819 | 4.910 | 0.010 |
| C16:1N7 | 13.516 | 11.342 | 13.932 | 13.158 | 12.273 | 11.446 | 0.532 | 0.589 |
| C17:0 | 0.714 | 0.689 | 0.641 | 0.628 | 0.577 | 0.564 | 3.273 | 0.043 |
| C17:1N7 | 1.062 | 0.939 | 0.807 | 0.814 | 0.873 | 0.843 | 2.038 | 0.137 |
| C18:0 | 56.347 | 47.203 | 47.645 | 45.315 | 40.140 | 41.039 | 6.244 | 0.003 |
| C18:1N9 | 62.658 | 41.994 | 47.061 | 43.025 | 42.914 | 42.835 | 2.747 | 0.070 |
| C18:1TN9 | 0.408 | 0.286 | 0.237 | 0.201 | 0.308 | 0.201 | 2.578 | 0.082 |
| C18:2N6 | 137.475 | 100.765 | 103.635 | 97.846 | 94.803 | 86.513 | 3.883 | 0.024 |
| C18:2TTN6 | 0.238 | 0.174 | 0.179 | 0.150 | 0.184 | 0.152 | 1.070 | 0.348 |
| C18:3N3 | 1.246 | 1.030 | 1.183 | 1.027 | 0.954 | 0.916 | 1.716 | 0.186 |
| C18:3N6 | 1.197 | 1.103 | 1.037 | 0.903 | 1.085 | 0.872 | 0.532 | 0.589 |
| C20:0 | 1.127 | 0.571 | 0.351 | 0.290 | 0.278 | 0.234 | 9.337 | < 0.001 |
| C20:1N9 | 2.015 | 0.939 | 1.220 | 0.614 | 0.993 | 0.734 | 1.897 | 0.156 |
| C20:2N6 | 3.566 | 3.574 | 3.646 | 3.531 | 3.669 | 3.725 | 0.058 | 0.944 |

(continued)

| fatty acid | average value (THEM) | median (THEM) | average value (NS) | median (NS) | average value (comparison) | median (comparison) | F | P value |
|---|---|---|---|---|---|---|---|---|
| C20:3N3 | 0.395 | 0.337 | 0.566 | 0.540 | 0.371 | 0.347 | 12.85 2 | < 0.001 |
| C20:3N6 | 26.394 | 24.024 | 23.545 | 21.967 | 24.392 | 24.075 | 0.835 | 0.438 |
| C20:4N6 | 86.394 | 80.404 | 89.092 | 90.703 | 88.562 | 86.514 | 0.076 | 0.927 |
| C20:5N3 | 0.614 | 0.369 | 0.215 | 0.188 | 0.151 | 0.134 | 12.955 | < 0.001 |
| C21:0 | 0.142 | 0.116 | 0.076 | 0.073 | 0.074 | 0.077 | 8.440 | < 0.001 |
| C22:0 | 1.971 | 1.485 | 1.756 | 1.389 | 1.998 | 1.300 | 0.271 | 0.764 |
| C22:1N9 | 9.569 | 6.997 | 7.392 | 6.903 | 4.582 | 4.076 | 10.379 | < 0.001 |
| C22:2N6 | 3.750 | 3.511 | 3.430 | 3.184 | 3.060 | 2.880 | 0.861 | 0.426 |
| C22:4N6 | 3.571 | 3.325 | 3.658 | 3.605 | 3.707 | 3.581 | 0.097 | 0.908 |
| C22:5N3 | 2.323 | 1.862 | 2.067 | 1.813 | 2.134 | 1.560 | 0.311 | 0.734 |
| C22:5N6 | 3.560 | 3.487 | 3.819 | 3.828 | 4.100 | 3.522 | 0.897 | 0.412 |
| C22:6N3 | 6.370 | 6.034 | 6.090 | 5.329 | 6.246 | 6.023 | 0.100 | 0.905 |
| C23:0 | 0.171 | 0.107 | 0.081 | 0.070 | 0.051 | 0.040 | 12.731 | < 0.001 |
| C24:0 | 0.804 | 0.491 | 0.299 | 0.254 | 0.208 | 0.180 | 11.609 | < 0.001 |
| C24:1N9 | 27.289 | 25.613 | 27.672 | 25.543 | 28.701 | 25.588 | 0.151 | 0.860 |
| C6:0 | 1.268 | 1.033 | 1.084 | 1.060 | 1.402 | 1.300 | 2.277 | 0.109 |
| C8:0 | 0.088 | 0.037 | 0.057 | 0.053 | 0.028 | 0.022 | 4.760 | 0.011 |
| Total MUFA | 151.426 | 127.730 | 122.957 | 117.964 | 115.040 | 121.479 | 4.040 | 0.021 |
| Total_N3 | 10.948 | 9.555 | 10.122 | 9.088 | 9.856 | 9.007 | 0.505 | 0.605 |
| Total_N6 | 266.145 | 233.985 | 232.042 | 234.020 | 223.562 | 215.155 | 2.223 | 0.115 |
| Total_PUF A | 277.093 | 241.162 | 242.164 | 241.044 | 233.418 | 223.316 | 2.195 | 0.118 |
| Total_SFA | 75.678 | 172.089 | 152.517 | 146.986 | 132.569 | 139.578 | 5.916 | 0.004 |

(3) SNK-q test: For the medium and long-chain fatty acids with significant differences among the three groups by variance analysis, the SNK-q test was used to make pairwise comparisons among the three groups. The results showed that compared with the control group, the IH group had significant differences ($P<0.05$) in 17 medium and long-chain fatty acids, namely: C10:0, C11:0, C12:0, C14:0, C15:0, C15:1N5, C16:0, C17:0 , C18:0, C18:2N6, C20:0, C20:5N3, C21:0, C22:1N9, C23:0, C24:0, and C8:0,And there are also significant differences in total saturated fatty acids (SFA) and total monounsaturated fatty acids (MUFA) ($P<0.05$);IH group compared with the NS group, there were 13 medium and long-chain fatty acids with significant differences ($P<0.05$), respectively:C10:0, C11:0, C12:0, C15:1N5, C18:0, C18:2N6, C20:0, C20:3N3, C20:5N3, C21:0, C22:1N9, C23:0 and C24: 0,And there were also significant differences in total monounsaturated fatty acids (MUFA) (P<0.05); compared with the control group, there were significant differences in 3 medium and long-chain fatty acids (P<0.05), respectively: C15:0 , C20:3N3 and C22:1N9 (see Table 5).

(4) ROC curve analysis: draw the ROC curves of the above 44 differential metabolites, and calculate the AUC value. Compared with the control group, the AUC values of 12 medium and long-chain fatty acids in the IH group were greater than 0.7, namely: C10:0, C11:0, C15:1N5, C18:0, C20:0, C20:5N3, and C21:0 , C22:1N9, C23:0, C24:0, C6:0 and C8:0; compared with the IH group and the NS group, there were 9 medium and long-chain fatty acids with AUC values greater than 0.7, which were: C10:0, C12:0, C15:1N5, C20:0, C20:3N3, C20:5N3, C21:0, C23:0, and C24:0. (See Table 5)

**Table 5**

| fatty acid | F | P value | SNK-qtest (IH vs control) | SNK-qtest (IH vs NS) | SNK-qtest (NS vs control) | AUC (IH vs control) | AUC (IH vs NS) |
|---|---|---|---|---|---|---|---|
| C10:0 | 6.876 | 0.002 | P<0.05 | P<0.05 | | 0.785 | 0.707 |
| C11:0 | 8.735 | 0.000 | P<0.05 | P<0.05 | | 0.774 | 0.690 |
| C12:0 | 4.351 | 0.016 | P<0.05 | P<0.05 | | 0.691 | 0.731 |
| C13:0 | 2.598 | 0.080 | | | | 0.548 | 0.543 |
| C14:0 | 4.551 | 0.013 | P<0.05 | | | 0.686 | 0.580 |
| C14:1N5 | 2.539 | 0.085 | | | | 0.596 | 0.663 |
| C15:0 | 4.633 | 0.012 | P<0.05 | | P<0.05 | 0.697 | 0.539 |
| C15:1N5 | 7.170 | 0.001 | P<0.05 | P<0.05 | | 0.744 | 0.711 |
| C16:0 | 4.910 | 0.010 | P<0.05 | | | 0.673 | 0.572 |
| C16:1N7 | 0.532 | 0.589 | | | | 0.473 | 0.401 |
| C17:0 | 3.273 | 0.043 | P<0.05 | | | 0.662 | 0.575 |
| C17:1N7 | 2.038 | 0.137 | | | | 0.558 | 0.641 |
| C18:0 | 6.244 | 0.003 | P<0.05 | P<0.05 | | 0.704 | 0.569 |
| C18:1N9 | 2.747 | 0.070 | | | | 0.532 | 0.457 |
| C18:1TN9 | 2.578 | 0.082 | | | | 0.638 | 0.652 |
| C18:2N6 | 3.883 | 0.024 | P<0.05 | P<0.05 | | 0.670 | 0.582 |
| C18:2TTN6 | 1.070 | 0.348 | | | | 0.591 | 0.588 |
| C18:3N3 | 1.716 | 0.186 | | | | 0.623 | 0.495 |
| C18:3N6 | 0.532 | 0.589 | | | | 0.601 | 0.607 |
| C20:0 | 9.337 | 0.000 | P<0.05 | P<0.05 | | 0.808 | 0.705 |
| C20:1N9 | 1.897 | 0.156 | | | | 0.633 | 0.662 |
| C20:2N6 | 0.058 | 0.944 | | | | 0.455 | 0.482 |
| C20:3N3 | 12.852 | 0.000 | | P<0.05 | P<0.05 | 0.471 | 0.146 |
| C20:3N6 | 0.835 | 0.438 | | | | 0.532 | 0.584 |
| C20:4N6 | 0.076 | 0.927 | | | | 0.436 | 0.387 |
| C20:5N3 | 12.955 | 0.000 | P<0.05 | P<0.05 | | 0.849 | 0.718 |

(continued)

| fatty acid | F | P value | SNK-qtest (IH vs control) | SNK-qtest (IH vs NS) | SNK-qtest (NS vs control) | AUC (IH vs control) | AUC (IH vs NS) |
|---|---|---|---|---|---|---|---|
| C21:0 | 8.440 | 0.000 | P<0.05 | P<0.05 | | 0.747 | 0.736 |
| C22:0 | 0.271 | 0.764 | | | | 0.572 | 0.553 |
| C22:1N9 | 10.379 | 0.000 | P<0.05 | P<0.05 | P<0.05 | 0.827 | 0.546 |
| C22:2N6 | 0.861 | 0.426 | | | | 0.601 | 0.552 |
| C22:4N6 | 0.097 | 0.908 | | | | 0.455 | 0.430 |
| C22:5N3 | 0.311 | 0.734 | | | | 0.591 | 0.527 |
| C22:5N6 | 0.897 | 0.412 | | | | 0.428 | 0.426 |
| C22:6N3 | 0.100 | 0.905 | | | | 0.529 | 0.529 |
| C23:0 | 12.731 | 0.000 | P<0.05 | P<0.05 | | 0.849 | 0.705 |
| C24:0 | 11.609 | 0.000 | P<0.05 | P<0.05 | | 0.821 | 0.702 |
| C24:1N9 | 0.151 | 0.860 | | | | 0.486 | 0.472 |
| C6:0 | 2.277 | 0.109 | | | | 0.295 | 0.437 |
| C8:0 | 4.760 | 0.011 | P<0.05 | | | 0.713 | 0.418 |
| Total_MUF A | 4.040 | 0.021 | P<0.05 | P<0.05 | | 0.622 | 0.580 |
| Total_N3 | 0.505 | 0.605 | | | | 0.593 | 0.538 |
| Total_N6 | 2.223 | 0.115 | | | | 0.611 | 0.537 |
| Total_PUF A | 2.195 | 0.118 | | | | 0.607 | 0.541 |
| Total_SFA | 5.916 | 0.004 | P<0.05 | | | 0.684 | 0.583 |

(5) Binary logistic regression analysis of IH group VS control group: screening IH group and control group Different even-numbered carbon medium and long-chain fatty acids: C10:0, C12:0, C14:0, C16:0, C18:0, C18:2N6, C20:0, C20:5N3, C22:1N9, C24:0 and C8: 0, a total of 11, binary logistic regression analysis was carried out, and the optimal model was obtained by using the step-by-step method, and the optimal model constructed with 6 variables was established (Table 6 below). To calculate the new predicted value (Y): $Y=logit(P)=Ln(P/(1-P))= 126.02 \times C10:0 - 0.16 \times C16:0 + 5.38 \times C20:0 + 23.28 \times C20:5N3 + 0.78 \times C22:1N9 - 67.68 \times C8:0 + 1.79$

**Table 6:** Binary logistic regression of IH group VS control group

| fatty acid | B | standard error | p-value |
|---|---|---|---|
| C10:0 | 126.02 | 74.24 | 0.090 |
| C16:0 | -0.16 | 0.06 | 0.008 |
| C20:0 | 5.38 | 4.25 | 0.205 |
| C20:5N3 | 23.28 | 12.78 | 0.069 |
| C22:1N9 | 0.78 | 0.4 | 0.047 |
| C8:0 | -67.68 | 63.92 | 0.290 |
| constant | 1.79 | 2.28 | 0.432 |

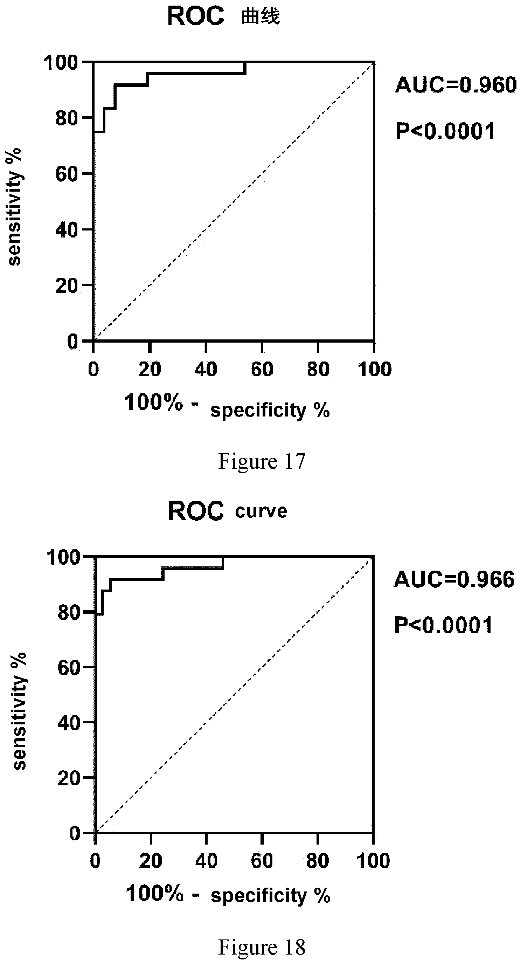

(6) ROC curve analysis of the fitting model: use this model to generate the predicted value Y value, and draw the ROC curve with the Y value (as shown in Figure 17), and calculate the AUC up to 0.960. At this time, the sensitivity of the model is 0.917 and the speciality is 0.923.

(7) Binary logistic regression analysis of IH group VS NS group:

Screening the difference between IH group and NS group even-numbered carbon long-chain fatty acids: C10:0, C12:0, C18:0, C18:2N6, C20:0, C20:3N3, C20:5N3, C22:1N9 and C24:0, A total of 9, binary logistic regression analysis was carried out, and the optimal model was obtained by stepwise method, and the optimal model constructed with 4 variables was established (Table 7 below).

Thus calculating the new predicted value (Y): **Y=logit(P)=Ln(P/(1-P))=12.41xC12:0-19.42xC20:3N3+14.10xC20:5N3-0.44xC 22:1N9+5.56**

Table 7: Binary logistic regression for IH group VS NS group

| fatty acid | B | standard error | p-value |
|---|---|---|---|
| C12:0 | 12.41 | 4.92 | 0.012 |
| C20:3N3 | -19.42 | 5.70 | 0.001 |
| C20:5N3 | 14.10 | 8.05 | 0.080 |
| C22:1N9 | -0.44 | 0.33 | 0.180 |
| constant | 5.56 | 2.48 | 0.025 |

(8) ROC curve analysis of the fitted model: the model was used to generate the predicted value Y, and the ROC curve was drawn with the Y value (Figure 18), and the calculated AUC was 0.966. At this time, the sensitivity of the model was 0.917, and the specificity was 0.946.

In conclusion, there are significant differences between the fatty acid indexes of infantile hemangioma and neonatal erythema with similar clinical appearance symptoms. The inventors of the present invention have found that there are not only significant differences between the fatty acid indexes of infantile hemangioma children and normal children, but also significant differences between the fatty acid indexes of neonatal erythema with similar clinical appearance symptoms, which is beneficial to clinically distinguish other diseases with symptoms similar to infantile hemangiomas, so as to avoid overtreatment or delayed treatment timing caused by misdiagnosis.

**Fatty acid group indicator Embodiment 2:**

According to the above fatty acid group index embodiment 1 method, further screening and verification of the IH detection effect by detecting a small amount of fatty acids. The differences from the above fatty acid group index embodiment 1 are highlighted below.

**Obtaining sample information**

A total of 63 patients with IH positive specimens were collected during follow-up, and 24 patients were randomly selected as the training set of the IH group. In addition, according to the infant gender, birth weight, gestational weeks and other relevant information of the children in the IH group, matching was conducted, and 26 sample control group training sets with the same conditions were screened. The test set is randomly selected with 28 cases as the IH group test set. In addition, according to the infant gender, birth weight, gestational weeks and other relevant information of the children in group IH, the matching conditions were used as matching conditions, and 32 samples of the control group with the same conditions were selected as the test set of the control group.

**Fatty acid test data were obtained**

The fatty acid data of the training set and the test set samples were measured by the above-mentioned fatty acid detection method.

In the training set and the test set, the total number of even carbon differential fatty acids (p<0.05) was 11, including cis-5, 8, 11, 14, 17-eicosapentaenoic acid, erucic acid, linoleic acid, stearic acid, cis-11-eicosapentaenoic acid, arachacid, capric acid, myristic acid, palmitic acid, decanoate, Myristate, palmitate, octanoate, tetracosanoate) (C20:5 n3, C22:1 the n9, C18:2 n6, C18:0, C20:1 n9, C20:0, C10:0, C14:0, C16:0, C8:0, C24: 0) as shown in **Table 8.**

**Table 8**

| EVEN-NUMBERED CARBON FATTY ACIDS WERE DIFFERENT IN BOTH BATCHES | | TRAINING SET DATA | | | |
|---|---|---|---|---|---|
| | | Multiple change | p- value (T test) | AUC | P(AUC) |
| zfs0001 | C10:0 | 2.05680931 | 0.00712709 | 0.7852564 | 0.0005473 |
| zfs0005 | C14:0 | 1.405472314 | 0.013531497 | 0.6858974 | 0.0242886 |
| zfs0009 | C16:0 | 1.280179 | 0.008605967 | 0.6730769 | 0.0359771 |
| zfs0013 | C18:0 | 1.403749634 | 0.003917408 | 0.7035256 | 0.013658 |
| zfs0016 | C18:2N6 | 1.450114664 | 0.03296498 | 0.6698718 | 0.0395566 |
| zfs0020 | C20:0 | 4.052589244 | 0.004999897 | 0.8076923 | 0.0001928 |
| zfs0021 | C20:1N9 | 2.028896504 | 0.034341765 | 0.6330128 | 0.1070215 |
| zfs0026 | C20:5N3 | 4.063899911 | 0.000820796 | 0.849359 | 2.304E-05 |
| zfs0029 | C22:1N9 | 2.088188606 | 0.000528246 | 0.8269231 | 7.453E-05 |
| zfs0036 | C24:0 | 3.861035025 | 0.001321701 | 0.8205128 | 0.0001029 |
| zfs0039 | C8:0 | 3.160951336 | 0.019706952 | 0.713141 | 0.0098046 |

**Continuation of Table 8**

| TEST SET DATA | | |
|---|---|---|
| Multiple change | p-value (T test) | AUC (A over B plus D) |
| 14.37978226 | 3.83076E-09 | 0.938 |
| 2.969710454 | 5.17568E-09 | 0.911 |
| 2.472324809 | 7.56839E-10 | 0.882 |
| 2.105599798 | 2.51299E-07 | 0.951 |
| 2.378743233 | 1.36996E-06 | 0.958 |
| 63.45552674 | 1.49574E-10 | 0.987 |
| 15.81861152 | 4.8717E-09 | 0.931 |
| 2.646920437 | 0.000140038 | 0.856 |
| 16.89111097 | 1.8511E-06 | 0.946 |
| 4.240622636 | 1.23929E-05 | 0.942 |
| 3.192145091 | 1.3846E-05 | 0.8 |

The above 11 even-carbon differential fatty acids are analyzed by binary logistics regression, and the optimal model is obtained by stepwise method. The optimal model is constructed with 5 variables, as shown in **Table 9.**

| Variable in an equation | | | | | | | |
|---|---|---|---|---|---|---|---|
| | | B | Standard error | Wald | Degree of freedom | significance | Exp (8) |
| Step 1 | C10:0 | 66.062 | 49. 852 | 1.756 | 1 | 0.185 | 4902892411507649 00 00000000000.000 |
| | C16:0 | -0.158 | 0.062 | 6.443 | 1 | 0.011 | 0.854 |
| | C20:0 | 4.560 | 4.418 | 1.065 | 1 | 0.302 | 95.562 |
| | C20:5N3 | 24.205 | 13.250 | 3.337 | 1 | 0.068 | 32517540987.178 |
| | C20:5N3 | 0.814 | 0.410 | 3.930 | 1 | 0.047 | 2.256 |
| | constant | 1.189 | 2.093 | 0.322 | 1 | 0.057 | 3.283 |
| a. Enter variables in Step 1: zfs0001, zfs0009, zfs0020, zfs0026, zfs0029. | | | | | | | |

**Thus, the new predicted value (Y) is calculated:**

$$\text{logit(P)=Ln(P/(1-P))}=66.062\times \text{C10:0}-0.158\times \text{C16:0}+4.560\times \text{C20:0}+24.205$$

$$\times \text{C20:5N3}+0.814\times \text{C22:1N9}+1.189$$

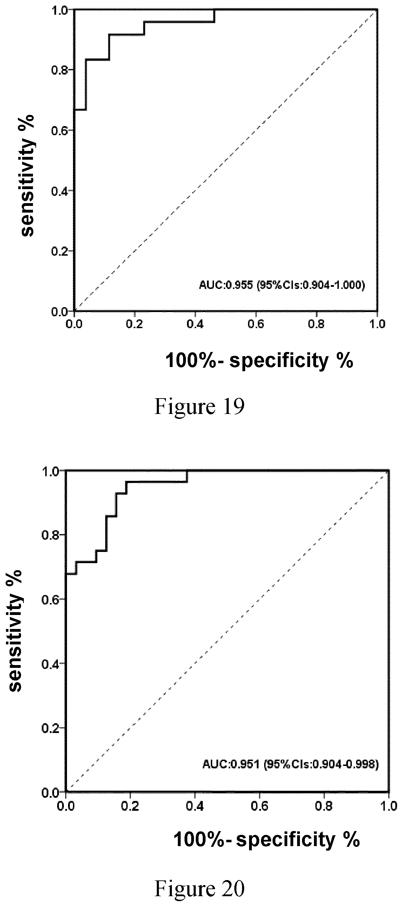

ROC curve analysis of fitted model:
The predicted Y value was generated by this model, and the ROC curve was drawn with the Y value (as shown in FIG. 19 and FIG. 20), and the AUC of the training set and the test set were calculated to be 0.955 and 0.951.
From the above results, it can be seen that even if only 5 kinds of fatty acids are detected, good detection effect can be obtained.

**Metabolome Examples of indicators:**

**(1) Experimental method**

1. Sample treatment: Take out the samples (all samples shown in Table 2) at -80°C, slowly dissolve at 4°C, take 100ul of samples from each group, add 400ul of pre-cooled methanol-acetonitrile solution (1:1, v/v), Vortex for 60s, place at -20°C for 1h to precipitate protein, centrifuge at 14000rcf, 4°C for 20min, take the supernatant and freeze-dry, store the sample at -80°C.
2. Sample detection: The sample was separated by Agilent 1290 Infinity LC ultra-high performance liquid chromatography (UHPLC) HILIC column. After the sample detection, the AB Triple TOF 6600 mass spectrometer was used to collect the first-order and second-order spectra of the sample.
3. Data processing: Metabolome detection data was converted into .mzXML format by ProteoWizard, and then XCMS program was used for peak alignment, retention time correction and peak area extraction. The identification of metabolite structure was carried out by accurate mass matching (<25 ppm) and secondary spectrum matching, and the self-built database of the laboratory was searched. XCMS software was used to extract metabolite ion peaks, and 10733 and 9688 peaks were detected in positive and negative ion modes, respectively. And through the laboratory self-built database for qualitative, positive ion mode, negative ion mode (positive ion mode, negative ion mode) qualitative metabolites 228,189 metabolites respectively.

**Table 10**

| model | Number of peaks | qualitative metabolites |
|---|---|---|
| Positive ions | 10733 | 228 |
| negative ions | 9688 | 189 |

4. Statistics:

Data analysis was performed using SPSS software (version 25.0; IBM, Armonk NY, USA) and SIMCA-P software (version 14.1; Umetrics, Umeå, Sweden). Two independent samples T-test (Student's t-test) was used to analyze whether the variables were different, and the two-tailed test P value less than 0.05 was considered significant. Orthogonal partial least squares discriminant analysis (OPLS-DA) is used to establish a relationship model between the expression of metabolites and the sample category to realize the prediction of the sample category, and at the same time calculate the variable importance for the projection (Variable Importance for the Projection, VIP) to measure the effect of the expression pattern of each metabolite on The impact strength and explanatory power of the classification and discrimination of samples in each group can assist in the screening of marker metabolites (with VIP >1.0 as the screening standard). ROC (receiver operating characteristic curve) analysis was used to judge the diagnostic value of difference variables. Binary logistic regression analysis was used to construct a logistic regression model to further improve the predictive sensitivity and specificity, and improve the diagnostic value.

**(2) Results:**

(1) Analysis of differential metabolites: Metabolites with both orthogonal partial least squares discriminant analysis VIP >1 and T-test analysis P < 0.05 were selected as metabolites with significant differences. There were 21 metabolites with significant difference in positive ion mode, and 21 metabolites with significant difference in negative ion mode.
(2) ROC curve analysis: draw the ROC curves of the above 42 differential metabolites, and calculate the AUC value. Among them, the AUC values of 12 differential metabolites were greater than 0.7, and the AUC values of 7 differential metabolites were greater than 0.75. See Table 11 below for details.

**Table 11:** Differential metabolites

| describe | median (THEM) | average value (THEM) | S.D. (THEM) | median (compar ison) | average value (compa rison) | S.D. (compar ison) | VI P | T-test (P value) | AUC |
|---|---|---|---|---|---|---|---|---|---|
| positive ion mode (3-carboxyprop yl) trimethylam monium cation | 318829 | 337322 | 94154 | 268980 | 290309 | 75926 | 1.809 | 0.037 | 0.671 |
| α-D-( )-talose | 16412 | 23794 | 21161 | 14509 | 15218 | 4831 | 1.266 | 0.030 | 0.641 |
| 1-Aminocyclop ropanecarboxyl ic acid | 78140 | 85632 | 33244 | 62309 | 63625 | 13650 | 1.212 | 0.001 | 0.723 |
| 1-myristoyl-sn-glycero-3-phosp hocholine | 204125 | 243120 | 122191 | 152627 | 176533 | 86010 | 2.129 | 0.017 | 0.756 |
| 1-oleoyl-sn-glyc ero-3-phosphoc holine | 51623 | 72145 | 48799 | 47835 | 52639 | 21808 | 1.671 | 0.046 | 0.606 |
| 1-palmitoyl-sn-glycero-3-phosp hocholine | 5485501 | 7466180 | 6888066 | 4577518 | 4507420 | 2206886 | 10.600 | 0.025 | 0.682 |
| 1-Stearoyl-sn-gl ycero-3-phosph ocholine | 98217 | 131318 | 111441 | 73130 | 87696 | 38256 | 2.721 | 0.042 | 0.634 |
| Bilirubin | 121286 | 182944 | 184129 | 103975 | 108052 | 32347 | 4.064 | 0.027 | 0.670 |
| choline | 155944 | 161306 | 55092 | 117173 | 123514 | 33338 | 1.681 | 0.002 | 0.729 |
| D-2-pipericolic acid | 393813 | 366494 | 154802 | 313629 | 289746 | 109651 | 2.554 | 0.029 | 0.720 |
| DL-indole-3-lac tic acid | 774455 | 807224 | 258688 | 584886 | 601723 | 201018 | 3.230 | 0.001 | 0.789 |
| Glycyl glutamate | 12840 | 17494 | 13646 | 6743 | 8584 | 7216 | 1.033 | 0.002 | 0.763 |
| Glycerophosph orylcholine | 1251462 | 1341116 | 614631 | 830505 | 961891 | 399832 | 5.393 | 0.006 | 0.694 |
| Isomaltose | 164450 | 232645 | 218406 | 140273 | 144117 | 46350 | 4.162 | 0.029 | 0.646 |
| L-Arginine | 4486781 | 4189379 | 1382270 | 4224622 | 3248590 | 1864913 | 6.779 | 0.032 | 0.634 |
| L-glutamic acid | 92028 | 108588 | 72200 | 43835 | 54623 | 34453 | 1.933 | <0.001 | 0.738 |
| L-phenylalanin e | 460866 | 491808 | 140133 | 409709 | 396129 | 73837 | 2.144 | 0.001 | 0.762 |
| L-tryptophan NG,NG-Dimeth | 154242 | 163224 | 50570 | 119779 | 120672 | 41031 | 1.460 | 0.001 | 0.794 |
| yl-L-arginine | 1578923 | 1720633 | 1051204 | 1200899 | 1226584 | 592838 | 4.751 | 0.026 | 0.669 |
| Tyramine | 1113942 | 1179400 | 290829 | 984725 | 962736 | 174741 | 3.366 | 0.001 | 0.750 |
| Phenylacetic acid | 22391 | 22557 | 5796 | 27750 | 28846 | 7451 | 1.033 | 0.001 | 0.756 |
| Anion mode (S)-Citromalic acid | 6167 | 11667 | 14554 | 5327 | 5643 | 2312 | 1.266 | 0.024 | 0.643 |
| 1-Oleoyl-L-α-ly sophosphatidic acid | 82402 | 112027 | 125362 | 58520 | 62618 | 36811 | 3.639 | 0.037 | 0.621 |
| 1-palmitoyl | 8580 | 17111 | 25773 | 4819 | 5915 | 4018 | 1.647 | 0.018 | 0.679 |
| lysophosphatidi c acid α-mangostin | 359331 | 376257 | 148392 | 316713 | 315394 | 79561 | 2.760 | 0.049 | 0.624 |
| Arachidonic Acid (peroxide free) | 368849 | 449317 | 252387 | 306437 | 345335 | 138980 | 3.686 | 0.049 | 0.604 |
| citric acid | 1575 | 10750 | 20950 | 1182 | 1744 | 2849 | 1.597 | 0.019 | 0.657 |

| describe | median (THEM ) | average value (THEM ) | S.D. (THEM ) | median (compar ison) | average value (compa rison) | S.D. (compar ison) | VI P | T-test (P value) | AUC |
|---|---|---|---|---|---|---|---|---|---|
| cyclamate | 26712 | 53820 | 65089 | 23137 | 25270 | 13771 | 1.070 | 0.019 | 0.617 |
| D-galactate | 18570 | 30921 | 26563 | 47558 | 55103 | 42388 | 2.099 | 0.012 | 0.695 |
| D-threitol | 210466 | 309909 | 297672 | 181664 | 194584 | 59441 | 4.225 | 0.036 | 0.605 |
| D(-)-beta-Hydr oxybutyrate | 90708 | 109747 | 75725 | 124524 | 168560 | 135681 | 2.688 | 0.047 | 0.602 |
| Inositol galactoside | 1042995 | 1544553 | 1349174 | 935864 | 1029118 | 395909 | 10.115 | 0.043 | 0.594 |
| glyceric acid | 64867 | 69975 | 33257 | 37110 | 39662 | 17540 | 2.351 | <0.001 | 0.809 |
| L-Aspartic Acid | 41762 | 62595 | 52823 | 29016 | 31032 | 14768 | 2.672 | 0.002 | 0.679 |
| L-Carnosine | 35521 | 41278 | 33837 | 56855 | 66084 | 40918 | 2.463 | 0.014 | 0.683 |
| L-malic acid | 16062 | 32591 | 38103 | 12548 | 15798 | 10828 | 2.077 | 0.020 | 0.648 |
| N-acetyl-L-asp artic acid | 27000 | 29910 | 13185 | 20684 | 21624 | 7228 | 1.238 | 0.003 | 0.694 |
| N-acetylneura minic acid | 33713 | 48444 | 36969 | 29165 | 32899 | 17857 | 1.920 | 0.039 | 0.679 |
| norethindrone acetate | 111580 | 132137 | 76908 | 89852 | 89933 | 41677 | 2.548 | 0.009 | 0.677 |
| Phosphocholine | 38592 | 46442 | 26464 | 27420 | 32016 | 15580 | 1.586 | 0.011 | 0.675 |
| sn-glycero-3-ph osphoethanola mine | 121230 | 153725 | 107756 | 78483 | 91679 | 44298 | 2.831 | 0.004 | 0.683 |
| Succinate | 28332 | 38221 | 23498 | 23040 | 26354 | 11530 | 1.921 | 0.014 | 0.627 |

(3) Binary logistic regression: Perform binary logistic regression analysis on the above 42 differential metabolites, and use a step-by-step algorithm to obtain the optimal model, and establish the optimal model constructed with 6 variables (see Table 10), so as to calculate New predicted value (Y): $Y = \text{logit}(P) = \ln(P/(1-P)) = -5.55 \times 10^{-5} \times \text{DL-indole-3-lactic acid} + 3.17 \times 10^{-4} \times \text{L-Tryptophan} + 3.50 \times 10^{-5} \times \text{cyclohexyl sulfamate} - 4.15 \times 10^{-5} \times \text{D-galactate} + 4.13 \times 10^{-5} \times \text{glyceric acid} + 1.45 \times 10^{-5} \times \text{sn-glycero-3-phosphoethanolamine} - 9.32$

**Table 12:** Logistic regression analysis to establish a predictive model.

| describe | B | standard error | significance |
|---|---|---|---|
| DL-indole-3-lactic acid | $-5.55 \times 10^{-5}$ | $2.68 \times 10^{-5}$ | 0.038 |
| L-Tryptophan | $3.17 \times 10^{-4}$ | $1.39 \times 10^{-4}$ | 0.022 |
| Cyclohexylsulfamate | $3.50 \times 10^{-5}$ | $1.77 \times 10^{-5}$ | 0.048 |
| D-galactate | $-4.15 \times 10^{-5}$ | $2.60 \times 10^{-5}$ | 0.111 |
| glyceric acid | $4.13 \times 10^{-5}$ | $2.77 \times 10^{-5}$ | 0.136 |
| sn-glycero-3-phosphoethanolamine | $1.45 \times 10^{-5}$ | $8.59 \times 10^{-6}$ | 0.091 |
| constant | $-9.32$ | 2.90 | 0.001 |

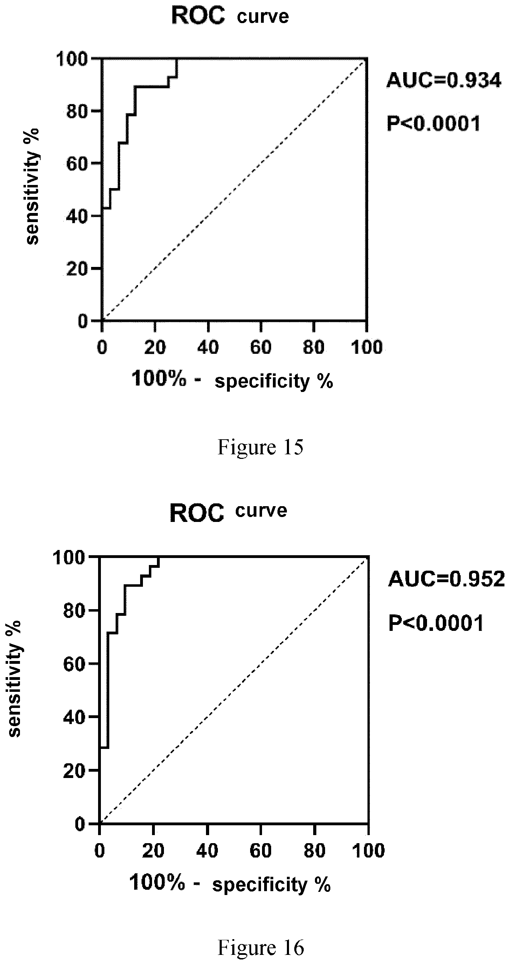

(4) Fitting model ROC curve analysis: use this model to generate the predicted value Y value, and draw the ROC curve with the Y value (as shown in Figure 16), and calculate the AUC up to 0.952. At this time, the sensitivity is 0.893 and the specificity is 0.906.

**Examples of proteomic indicators:**

**High-throughput swissport proteomics detection**

**(1) Experimental method**

1. Glossary

DDA: Data Dependent Acquisition, used in this article to establish a library in the DIA analysis process.

DIA: Data Independent Acquisition (Data Independent Acquisition), specifically refers to the data based on Thermo Scientific™ electrostatic field orbitrap Orbitrap in this articlenon-dependent collectionset.

Spectronaut Pulsar X: Proteomics software for the analysis of data independent acquisition (DIA) measurements. The well-known DIA data analysis software from Biognosys.

MaxQuant: The well-known proteomics software from Mann M.'s laboratory, used to build the library and use it when searching.

Direct DIA: The integrated database search engine called Pulsar enables spectral-library free DIA workflow (Integrated database search engine called Pulsar enabling spectral-library free DIA workflow). The algorithm from Biognosys can directly search the library for DIA data, which is used to increase the capacity of the library and increase the number of final qualitative and quantitative results of DIA.

**2. Protein detection:**

First, the combined samples with the same amount of samples were used as a pool, and Aglient-H14 separated the pool samples. The high-abundance and low-abundance components were respectively enzymolized, and the enzymolized peptides of the low-abundance group were graded by HPRP (10 parts). The above samples were analyzed by LC-MS/MS (Qe-Hf-dDA mode), and the proteome database was obtained, and the self-built database of APT was combined as the database of DIA.The specific steps are: carry out sample enzymatic hydrolysis and HPRP grading on the mixture of all samples, collect data dependent acquisition (DDA) mass spectrometry data, then use MaxQuant to search and identify, and use Spectronaut pulsar X software to construct a spectral library as a subsequent data independent acquisition ( DIA) database for quantification of protein profiles. Download the swissport data package to build a proteome database.

Each sample in the project was independently prepared for sample preparation and protein enzymatic hydrolysis, and then used for DIA (data independent acquisition) analysis on the machine. The obtained DIA original file is imported into Spectronaut Pulsar X for analysis, and the qualitative and quantitative protein expression data of each sample is obtained (the numerical value reflects the area under the peak curve of the protein, and the larger the value, the higher the protein content).

**3. Statistics:**

Data analysis was performed using SPSS software (version 25.0; IBM, Armonk NY, USA). Two independent samples T-test (Student's t-test) was used to analyze whether the variables were different, and the two-tailed test P value less than 0.05 was considered significant. ROC (receiver operating characteristic curve) was used to analyze the diagnostic value of differential variables. Binary logistic regression analysis was used to construct a logistic regression model to further improve the predictive sensitivity and specificity, and improve the diagnostic value.

**(2) Results**

(1) Protein screening: A total of 727 proteins were qualitatively quantified, and protein variables with missing values >50% in all samples were filtered to reduce the false positive rate. A total of 603 variables were screened. Missing values are filled with the mean value of variables in the same group to maintain the homogeneity within the data group;

(2) T-test: Two independent sample T-tests are performed on the selected proteins, and variables with a P value less than 0.05 are selected, a total of 47: PG.Genes:COLEC10, CPQ, LDHB, ACAN, KRT1, ARHGDIB, POSTN, LAMA5, CPN1, PROC, C1S, HPX, SUPT6H, BCAR3, BCHE, SOX30, C1R, PON1, CFHR4, PROZ, IGKV1D-16, APOC3, COMP, SELENOP, PMFBP1, GSN, SELL, FUCA2, HLA-B, FGFBP2, FKBP1A, HSPE1, SAA1, PGD, CDH1, SOD1, ISLR, CORO1A, ZYX, RLBP1, IDH1, IGKV6-21, SORL1, FAM3C, CST6, COTL1, and PIGR.

(3) ROC curve analysis: ROC analysis was performed on the above 47 proteins, and the AUC value was calculated. The results are shown in Table 13 below:

Table 13: Differential proteins.

| PG. Gene | Average (IH) | S.D.(IH) | mean (control) | S.D. (control) | multiple of difference | T-test (P value) | AUC |
|---|---|---|---|---|---|---|---|
| COLLECTION10 | 489637.08 | 764638.77 | 89623.20 | 164109.90 | 5.463 | 0.005 | 0.661 |
| CPQ | 36436.65 | 28262.77 | 21563.00 | 5069.75 | 1.690 | 0.005 | 0.713 |
| LDHB | 79562.26 | 83767.39 | 46791.54 | 14756.34 | 1.700 | 0.034 | 0.614 |
| not | 345978.53 | 667176.40 | 45809.12 | 26312.64 | 7.553 | 0.014 | 0.657 |
| KRT1 | 526992.48 | 490704.26 | 326165.09 | 245815.86 | 1.616 | 0.046 | 0.650 |
| ARHGDIB | 309369.77 | 421514.71 | 806844.88 | 839090.34 | 0.383 | 0.006 | 0.686 |
| POSTN | 77104.52 | 26098.29 | 156368.97 | 182303.19 | 0.493 | 0.026 | 0.656 |
| LAMA5 | 93517.72 | 59859.49 | 325520.40 | 444572.86 | 0.287 | 0.008 | 0.671 |
| CPN1 | 228304.42 | 55973.49 | 274373.92 | 64434.35 | 0.832 | 0.005 | 0.705 |
| PROC | 87150.44 | 31495.98 | 115149.00 | 47549.83 | 0.757 | 0.010 | 0.680 |
| C1S | 996392.59 | 191222.70 | 1111291.46 | 157926.85 | 0.897 | 0.013 | 0.680 |
| HPX | 3961113.56 | 1438689.00 | 5001838.30 | 1750057.41 | 0.792 | 0.016 | 0.646 |
| SUPT6H | 23247.95 | 11105.06 | 16716.20 | 9491.14 | 1.391 | 0.017 | 0.664 |
| BCAR3 | 390763.98 | 94742.67 | 309680.67 | 110582.61 | 1.262 | 0.004 | 0.731 |
| bche | 234913.11 | 57678.23 | 275137.19 | 68828.44 | 0.854 | 0.018 | 0.652 |
| SOX30 | 171068.60 | 68476.92 | 235378.31 | 92135.26 | 0.727 | 0.004 | 0.759 |
| C1R | 336921.53 | 59717.26 | 374292.27 | 63063.99 | 0.900 | 0.022 | 0.671 |
| MON1 | 904603.40 | 233372.19 | 1060586.34 | 275769.31 | 0.853 | 0.022 | 0.662 |
| CFHR4 | 121913.34 | 51559.66 | 89199.81 | 32542.13 | 1.367 | 0.004 | 0.733 |
| PROC | 69129.76 | 18268.32 | 86769.95 | 36231.60 | 0.797 | 0.023 | 0.657 |
| IGKVID-16 | 315793.03 | 82607.87 | 265469.33 | 85192.65 | 1.190 | 0.024 | 0.670 |
| APOC3 | 4917626.31 | 1267527.30 | 5734103.29 | 1446015.20 | 0.858 | 0.024 | 0.645 |
| COMP | 55980.44 | 13048.91 | 47845.23 | 15039.27 | 1.170 | 0.030 | 0.650 |
| SELENOP | 264281.79 | 111982.72 | 326773.69 | 109603.49 | 0.809 | 0.033 | 0.664 |
| PMFBP1 | 48075.43 | 9771.30 | 55789.43 | 17468.99 | 0.862 | 0.043 | 0.628 |
| GSN | 717081.40 | 125124.78 | 793469.40 | 161893.55 | 0.904 | 0.048 | 0.640 |
| SELL | 151157.85 | 36550.70 | 183022.22 | 76129.37 | 0.826 | 0.048 | 0.661 |
| FUCA2 | 52538.48 | 46157.10 | 121781.38 | 149762.35 | 0.431 | 0.022 | 0.683 |
| HLA-B | 10646604.77 | 2221792.49 | 9556245.15 | 1871595.86 | 1.114 | 0.044 | 0.688 |
| FGFBP2 | 59657.01 | 89861.92 | 25974.82 | 9987.49 | 2.297 | 0.039 | 0.667 |
| FKBP1A | 26359.98 | 8534.48 | 34358.27 | 8983.43 | 0.767 | 0.001 | 0.769 |
| HSPE1 | 3387870.63 | 4905644.02 | 7279872.42 | 5294606.09 | 0.465 | 0.005 | 0.756 |
| SAA1 | 34863.23 | 14180.22 | 138707.32 | 203188.86 | 0.251 | 0.009 | 0.627 |

| PG. Gene | Average (IH) | S.D.(IH) | mean (control) | S.D.(control) | multiple of difference | T-test (P value) | AUC |
|---|---|---|---|---|---|---|---|
| PGD | 189190.75 | 136856.42 | 92360.25 | 80392.91 | 2.048 | 0.001 | 0.743 |
| CDH1 | 46609.30 | 18911.42 | 79520.77 | 83116.28 | 0.586 | 0.045 | 0.771 |
| SOD1 | 68739.73 | 49231.43 | 48647.85 | 23658.30 | 1.413 | 0.044 | 0.645 |
| ISLR | 19732.98 | 8273.89 | 55587.07 | 81752.55 | 0.355 | 0.025 | 0.767 |
| CORO1A | 50342.75 | 29586.56 | 81949.86 | 56366.82 | 0.614 | 0.010 | 0.693 |
| ZYX | 83783.48 | 32466.72 | 108730.39 | 55576.88 | 0.771 | 0.042 | 0.664 |
| RLBP1 | 293537.94 | 76649.40 | 234020.76 | 70520.16 | 1.254 | 0.003 | 0.771 |
| IDH1 | 55569.85 | 55549.76 | 21911.64 | 24115.91 | 2.536 | 0.003 | 0.761 |
| IGKV6-21 | 345471.18 | 205800.45 | 245868.70 | 118661.96 | 1.405 | 0.023 | 0.703 |
| SORL1 | 4263013.59 | 2674588.87 | 3013720.32 | 2095660.79 | 1.415 | 0.047 | 0.704 |
| FAM3C | 11135.84 | 2445.34 | 15392.99 | 7456.62 | 0.723 | 0.005 | 0.747 |
| CST6 | 36577.08 | 13747.43 | 47662.60 | 20321.70 | 0.767 | 0.018 | 0.720 |
| COTL1 | 84724.49 | 55473.51 | 113931.08 | 55966.21 | 0.744 | 0.047 | 0.757 |
| PIGR | 812006.80 | 1523374.71 | 209093.87 | 345660.72 | 3.883 | 0.033 | 0.749 |

(4) Binary logistic regression analysis:For the above 47 proteins, binary logistic regression analysis was carried out, and the optimal model was obtained by stepwise algorithm, and the optimal model constructed with 6 variables was established (see Table 12), so as to calculate the new predicted value (Y): $Y=\text{logit }(P)=\text{Ln}(P/(1-P))= 7.02\times10^{-6}\times\text{KRT1} - 4.09\times10^{-5}\times\text{POSTN} - 1.18\times10^{-4}\times\text{PROC} - 1.06\times10^{-4}\times\text{PROZ}-2.30\times10^{-4}\times\text{PNWBP1}-1.12\times10^{-4}\times\text{SELL}+51.03$

Table 14: Logistic regression analysis to establish a predictive model.

| PG gene | B | standard error | P value |
|---|---|---|---|
| KRT1 | $7.02\times10^{-6}$ | $3.82\times10^{-6}$ | 0.066 |
| POSTN | $-4.09\times10^{-5}$ | $2.67\times10^{-5}$ | 0.126 |
| PROC | $-1.18\times10^{-4}$ | $5.13\times10^{-5}$ | 0.022 |
| PROC | $-1.06\times10^{-4}$ | $4.39\times10^{-5}$ | 0.016 |
| PMFBP1 | $-2.30\times10^{-4}$ | $9.04\times10^{-5}$ | 0.011 |
| SELL | $-1.12\times10^{-4}$ | $4.70\times10^{-5}$ | 0.017 |
| constant | -51.03 | 20.42 | 0.012 |

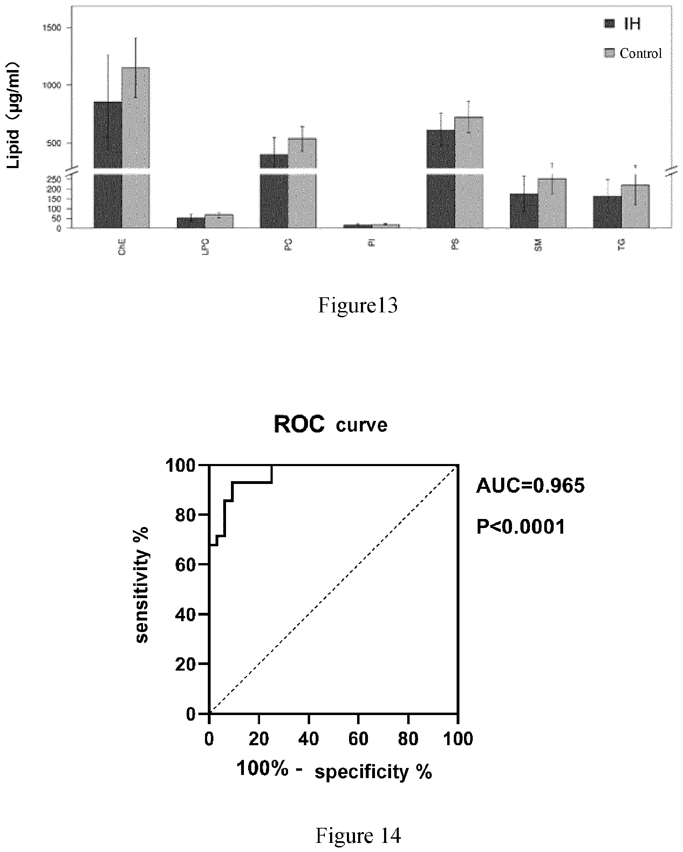

(5) Fitting model ROC curve analysis:Use this model to generate the predicted value Y value, and draw the ROC curve with the Y value (as shown in Figure 14), and calculate the AUC up to 0.965, at this time the sensitivity is 0.929, and the specificity is 0.906.

**High-throughput uniport proteomics detection**

**The difference from the above-mentioned high-throughput swissport proteomics detection is:**

1. Protein detection:

First of all, the combined samples with the same amount of samples to be detected were used as pool, and the pool samples were separated by Aglient-H14. The high-abundance and low-abundance components were respectively enzymolized, and the enzymolized peptides of the low-abundance group were graded by HPRP (10 parts). The above samples were analyzed by LC-MS/MS (Qe-Hf-dDA mode), and the proteome database was obtained, and the self-built database of APT was combined as the database of DIA. The specific steps are: carry out sample enzymatic hydrolysis and HPRP grading on the mixture of all samples, collect data dependent acquisition (DDA) mass spectrometry data, then use MaxQuant to search and identify, and use Spectronaut pulsar X software to construct a spectral library as a subsequent data independent acquisition (DIA) Database for the quantification of protein profiles. Download the uniport package to build a proteome database.

Each sample in the project was independently prepared for sample preparation and protein enzymatic hydrolysis, and then used for DIA (data independent acquisition) analysis on the machine. The obtained DIA original file was imported into Spectronaut Pulsar X for analysis, and the qualitative and quantitative protein quantity of each sample was obtained. (The numerical value reflects the area under the chromatographic peak curve of the protein. The larger the numerical value, the higher the protein content). A total of 2572 proteins were qualitatively quantified, of which 1672 proteins were detected in more than 80% of the samples.

**2. Results**

(1) Protein screening: A total of 2572 proteins were qualitatively quantified, and protein variables with missing values >20% in all samples were filtered to reduce the false positive rate. A total of 1672 variables were screened. The missing value is filled with the median value of the variable in the same group to maintain the homogeneity within the data group;
(2) T test: For the selected data, two independent sample T tests were used to select variables with a P value less than 0.05, a total of 71 variables. Immunoglobulins were further removed to obtain 15 differential proteins, PG.Genes: A0A1W6IYJ2, PRDX1, CAT, CPN1, APOC3, HPX, PROS1, CD5L, HMFT1766, C1QA, KRT1, BCHE, KRT2, APP and FGFBP2.
(3) ROC curve analysis: ROC curves were drawn for the above 15 variables, and AUC was calculated, as shown in Table 15:

EP 4 306 960 A1

Table 15

| PG gene | median (THEM) | average value (THEM) | S.D. (THEM) | median (control) | average value (control) | S.D. (control) | multi ple of diffe renc e | T-test (P value) | AUC |
|---|---|---|---|---|---|---|---|---|---|
| A0A1W6 IYJ2 | 614676.563 | 847905.466 | 782436.867 | 267682.690 | 449783.551 | 424430.888 | 1.885 | 0.016 | 0.621 |
| PRDX1 | 234488.560 | 380525.480 | 402208.233 | 195289.940 | 211431.779 | 108808.367 | 1.800 | 0.026 | 0.615 |
| CAT | 78874.492 | 228331.400 | 278356.728 | 511486.219 | 541221.860 | 518190.671 | 0.422 | 0.006 | 0.680 |
| CPN1 | 257049.453 | 246976.689 | 55755.876 | 292843.563 | 289185.742 | 59845.223 | 0.854 | 0.007 | 0.693 |
| APOC3 | 5772071.500 | 5645160.777 | 1421442.818 | 6292647.750 | 6559630.852 | 1679778.529 | 0.861 | 0.028 | 0.638 |
| HPX | 2910172.875 | 2866978.763 | 921713.682 | 3162924.125 | 3476699.133 | 1092386.498 | 0.825 | 0.024 | 0.643 |
| PROS1 | 296606.938 | 286566.921 | 59902.719 | 302783.891 | 341926.284 | 105979.206 | 0.838 | 0.018 | 0.642 |
| CD5L | 173409.594 | 194614.525 | 59632.287 | 220595.852 | 259493.933 | 137125.243 | 0.750 | 0.024 | 0.680 |
| HMFT1766 | 513888.453 | 525761.571 | 137879.794 | 576627.344 | 661163.935 | 300058.056 | 0.795 | 0.032 | 0.650 |
| C1QA | 1347714.688 | 1382551.732 | 297400.801 | 1138744.750 | 1178767.666 | 316600.309 | 1.173 | 0.013 | 0.663 |
| KRT1 | 392435.797 | 541165.941 | 502435.036 | 270476.336 | 320891.082 | 206787.318 | 1.686 | 0.027 | 0.642 |
| bche | 199200.516 | 201879.781 | 54047.997 | 218039.750 | 230290.208 | 54240.260 | 0.877 | 0.047 | 0.637 |
| KRT2 | 41263.826 | 59997.162 | 60285.469 | 34441.770 | 37347.588 | 16469.441 | 1.606 | 0.046 | 0.617 |
| APP | 22074.400 | 33219.803 | 33251.508 | 19111.795 | 20108.368 | 9528.227 | 1.652 | 0.037 | 0.641 |
| FGFBP2 | 29877.820 | 82835.041 | 117005.447 | 26050.620 | 32308.263 | 28825.976 | 2.564 | 0.021 | 0.617 |

(4) Binary logistic regression analysis: Perform binary logistic regression analysis on the above 15 proteins, and use a step-by-step algorithm to obtain the optimal model, and establish the optimal model constructed with 6 variables(See Table 14 below), so as to calculate the new predicted value (Y): $Y=logit(P)=Ln(P/(1-P))=3.96\times10^{-6}\times A0A1W6IYJ2 - 3.22\times10^{-6}\times CAT - 1.92\times10^{-5}\times PROS1 - 4.10\times10^{-5}\times CD5L + 3.36\times10^{-6}\times C1QA + 4.74\times10^{-6}\times KRT1 + 6.81$

**Table 16**

| PG. Gene | B | standard error | P value |
|---|---|---|---|
| A0A1W6IYJ2 | $3.96\times10^{6}$ | $1.43\times10^{-6}$ | 0.00549617 |
| CAT | $-3.22\times10^{-6}$ | $1.41\times10^{-6}$ | 0.02221397 |
| PROS1 | $-1.92\times10^{-5}$ | $8.15\times10^{6}$ | 0.01848923 |
| CD5L | $-4.10*10^{-5}$ | $1.54\times10^{-5}$ | 0.00786287 |
| C1QA | $3.36\times10^{-6}$ | $1.79\times10^{-6}$ | 0.0602534 |
| KRT1 | $4.74\times10^{-6}$ | $2.12\times10^{-6}$ | 0.02552159 |
| constant | 6.81 | 3.57 | 0.05647654 |

(5) ROC curve analysis of the fitting model: use this model to generate the predicted value Y value, and draw the ROC curve with the Y value (as shown in Figure 15), and calculate the AUC up to 0.934. At this time, the sensitivity is 0.893 and the specificity is 0.875.

**Examples of lipidome indicators:**

**1. Experimental equipment and reagents**

Q-Exactive Plus mass spectrometer (Thermo Scientific)
UHPLC Nexera LC-30A Ultra High Performance Liquid Chromatograph (SHIMADZU)
Low-temperature high-speed centrifuge (Eppendorf 5430R)
Column: Waters, ACQUITY UPLC CSH C18, 1.7 μm, 2.1 mm× 100 mm column
Acetonitrile (Thermo Fisher), Isopropanol (Thermo Fisher), Methanol (Thermo Fisher)
13 isotopic internal standards (Cer, LPC, PC, LPE, PE, PI, PS, PA, PG, SM, Chol Ester, DG, TG).

**2. Experimental method**

**2.1 Sample information**

Preparation of quality control samples (QC): Equal amounts of samples from each group were mixed for QC. QC samples are not only used to test the state of the instrument before sample injection and to balance the chromatography-mass spectrometry system, but also interspersed during the detection of samples to be tested to evaluate the system stability during the entire experiment.

**2.2 Sample preprocessing method**

Take 60ul of each sample, add 200 μL water and 20 μL internal lipid standard mixture, vortex mix, add 800 μL methyl tert-butyl ether (MTBE), vortex mix, add 240 μL pre-cooled Methanol, vortexed, sonicated in a low-temperature water bath for 20 minutes, placed at room temperature for 30 minutes, centrifuged at 14,000 g at 10°C for 15 minutes, took the upper organic phase, dried with nitrogen, and added 200 μL of 90% isopropanol/acetonitrile solution for mass spectrometry analysis. Vortex well, take 90 μL of complex solution, centrifuge at 14,000 g at 10°C for 15 min, and take the supernatant for analysis.

**2.3 Chromatography-mass spectrometry**

**2.3.1 Chromatographic conditions**

The samples were separated by UHPLC Nexera LC-30A ultra-high performance liquid chromatography system. C18 chromatographic column;

The column temperature is 45°C; the flow rate is 300 μL/min. Mobile phase composition A: Acetonitrile aqueous solution (acetonitrile: water = 6:4, v/v), B: acetonitrile isopropanol solution (acetonitrile: isopropanol = 1:9, v/v). The gradient elution program is as follows: 0 --- 2 min, B is maintained at 30%; 2 --- 25 min, B is linearly changed from 30% to 100%; 25 --- 35 min, B is maintained at 30%.

### 2.3.2 Mass Spectrometry Conditions

Electrospray ionization (ESI) positive and negative ion modes were used for detection. Samples were separated by UHPLC and analyzed by mass spectrometry using a Q Exactive series mass spectrometer (Thermo Scientific™). The ESI source conditions were as follows:
Heater Temp 300°C, Sheath Gas Flow rate 45 arb, Aux Gas Flow Rate 15 arb, Sweep Gas Flow Rate 1 arb, spray voltage (spray voltage) 3.0KV, capillary temperature (Capillary Temp) 350 °C, ion lens voltage frequency level (S-Lens RF Level) 50%, MS 1 scan ranges (scan ranges): 200-1800.
The mass-to-charge ratios of lipid molecules and lipid fragments were collected as follows: 10 fragment spectra (MS 2 scan, HCD) were collected after each full scan. MS 1 has a resolution of 70,000 at M/Z 200 and MS 2 has a resolution of 17,500 at M/Z 200.

### 2.4 Data processing and software setting

Use LipidSearch to perform peak identification, peak extraction, and lipid identification (secondary identification) on lipid molecules and internal standard lipid molecules. The main parameters are: precursor tolerance: 5 ppm, product tolerance: 5 ppm, product ion threshold: 5%.

### 2.5 Statistical analysis

SPSS software (version25.0; IBM, Armonk NY, USA) was used. Two independent samples T-test (Student's t-test) was used to analyze whether the variables were different, and the two-tailed test P value less than 0.05 was considered significant. The difference was calculated by calculating the Flod change (FC) value, and FC>1.5 or FC<0.67 were considered significant differences. ROC (receiver operating characteristic curve) analysis was used to analyze the diagnostic value of differential variables.

### 3. Results

### 3.1 Identification quantity statistics

The International Lipid Classification and Nomenclature Committee divides lipid compounds into 8 major types, each type can be divided into different subclasses (lipid class) according to the difference of the polar head, each subclass Classes are divided into different molecules (lipid species) according to differences such as carbon chain saturation or length, thus forming a three-level classification of lipid compounds: class-subclass-molecule.
The number of lipid compounds in the samples identified by positive and negative ion modes in this experiment.

**Table 17:** Number of lipid subclasses and molecular identifications

| Lipid subclass (Lipid class) | Lipid species |
|---|---|
| 25 | 821 |

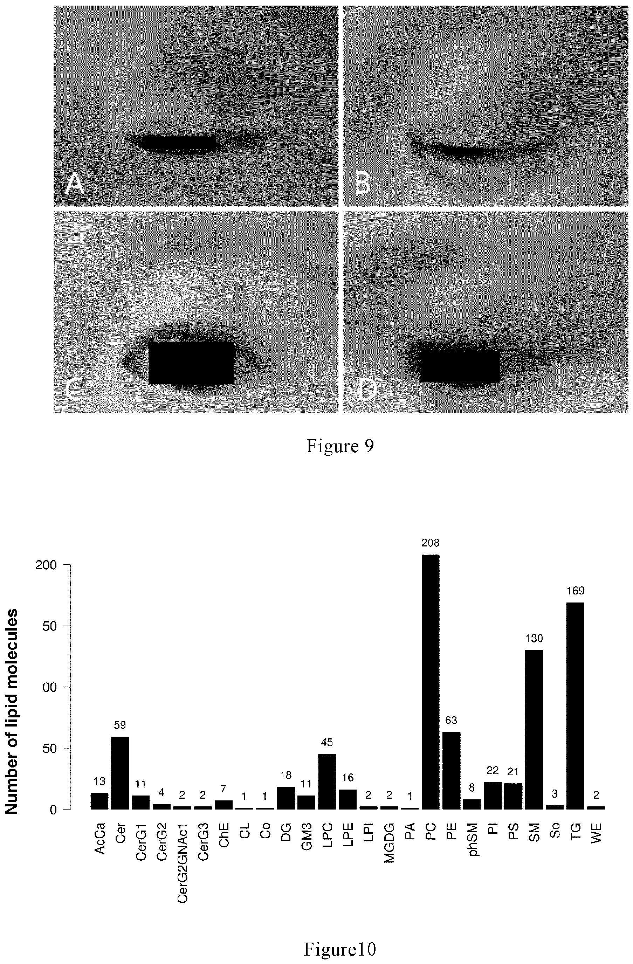

The statistical results of lipid subclasses (lipid class) identified in positive and negative ion modes and the number of lipid molecules (lipid species) identified in each category are shown in Table 1 and Figure 10.

### 3.2 Lipid difference analysis

### 3.2.1 Overall level

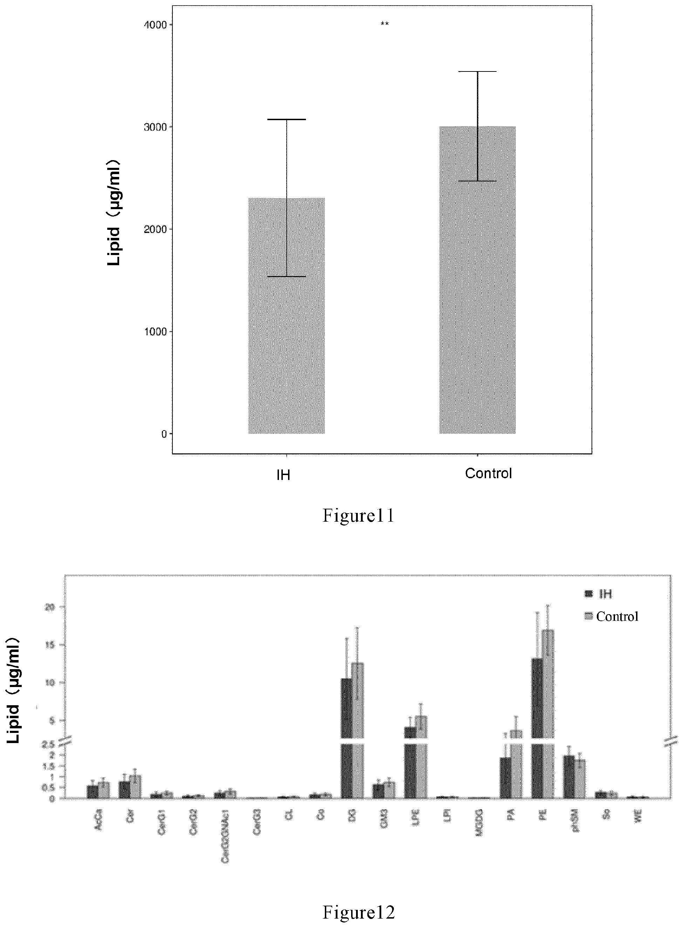

The contents of all the quantified lipid molecules in the same sample are summed, that is, the total lipid molecule content of the sample. Then the total content of samples from different groups can be compared, and the T test results showed that the total lipid content of the IH group was lower than that of the control group (P<0.01). (See Figure 11)

### 3.2.2 Subcategory level

The contents of all the quantified uniform subclasses of lipid molecules in the same sample were summed to compare the expression changes of lipid subclasses in different samples. The results of the T-test show thatAcCa, Cer, CerG1, CerG2, CerG2GNAc1, CerG3, ChE, CL, GM3, LPC, LPE, MGDG, PA, PC, PE, phSM, PI, PS, SM, and TGThere was a difference in the content between the two groups (P <0.05), the content of phSM in the IH group was higher than that in the control group, and the rest were lower than that in the control group. The content of Co, DG, LPI, So, WE had no significant difference between the two groups (*P* >0.05) (see Table 18, Figure 12 and Figure 13).

The diagnostic value of each differential lipid subclass was further analyzed by ROC, and the AUC value was calculated. The results showed that the AUC values of Cer, ChE, LPC, LPE, PA, PC, PE, PI, PS and SM were greater than 0.700, and the AUC value of PA was greater than 0.8, which had good diagnostic value (see Table 18).

**Table 18:** Differences in the expression of different subclasses of lipids between the two groups

| category | average value THEM | average value control | SD THEM | SD control | multiple of difference | P value | AUC |
|---|---|---|---|---|---|---|---|
| AcCa | 0.566 | 0.730 | 0.257 | 0.202 | 0.776 | 0.008 | 0.683 |
| Cer | 0.767 | 1.043 | 0.347 | 0.314 | 0.735 | 0.002 | 0.728 |
| CerG1 | 0.187 | 0.251 | 0.101 | 0.083 | 0.746 | 0.010 | 0.688 |
| CerG2 | 0.105 | 0.131 | 0.052 | 0.039 | 0.803 | 0.031 | 0.692 |
| CerG2GNAc1 | 0.245 | 0.318 | 0.127 | 0.120 | 0.772 | 0.027 | 0.662 |
| CerG3 | 0.014 | 0.018 | 0.009 | 0.006 | 0.779 | 0.040 | 0.655 |
| That | 851.707 | 1148.962 | 409.562 | 256.497 | 0.741 | 0.001 | 0.702 |
| CL | 0.068 | 0.084 | 0.028 | 0.019 | 0.807 | 0.010 | 0.682 |
| Co | 0.171 | 0.188 | 0.066 | 0.053 | 0.908 | 0.265 | 0.573 |
| DG | 10.493 | 12.532 | 5.347 | 4.702 | 0.837 | 0.121 | 0.633 |
| GM3 | 0.621 | 0.739 | 0.222 | 0.197 | 0.839 | 0.032 | 0.642 |
| LPC | 51.859 | 66.396 | 17.496 | 12.767 | 0.781 | <0.001 | 0.752 |
| LPE | 4.062 | 5.539 | 1.325 | 1.641 | 0.733 | <0.001 | 0.758 |
| LPI | 0.075 | 0.078 | 0.017 | 0.016 | 0.968 | 0.571 | 0.557 |
| MGDG | 0.022 | 0.028 | 0.009 | 0.009 | 0.808 | 0.023 | 0.654 |
| Well | 1.845 | 3.703 | 1.451 | 1.791 | 0.498 | <0.001 | 0.809 |
| PC | 399.514 | 535.430 | 147.477 | 106.452 | 0.746 | <0.001 | 0.752 |
| ON | 13.120 | 16.880 | 6.148 | 3.279 | 0.777 | 0.004 | 0.737 |
| phSM | 1.958 | 1.748 | 0.435 | 0.317 | 1.120 | 0.035 | 0.623 |
| PI | 16.084 | 18.904 | 3.639 | 2.774 | 0.851 | 0.001 | 0.733 |
| PS | 610.154 | 721.305 | 144.829 | 134.417 | 0.846 | 0.003 | 0.728 |
| SM | 174.300 | 251.434 | 90.046 | 70.220 | 0.693 | <0.001 | 0.733 |
| So | 0.269 | 0.237 | 0.091 | 0.082 | 1.139 | 0.145 | 0.608 |
| TG | 164.301 | 219.492 | 84.097 | 84.061 | 0.749 | 0.014 | 0.681 |
| WE | 0.072 | 0.064 | 0.040 | 0.032 | 1.122 | 0.410 | 0.542 |

### 3.2.3 Lipid molecular level

The qualitative and quantitative 821 lipid molecules between the two groups were subjected to two-sample T-test, and the results showed that there were differences in 567 lipid molecules (P<0.05), and the fold change (FC) was further screened to meet the difference of FC > 1.5. There are 6 molecules (FC>1.5) and 130 with FC<0.67. Finally, with the T-test P value <0.05, and FC>1.5 or FC<0.67 as the screening conditions, a total of 136 differential molecules were screened.

The diagnostic value of each differential molecule was further analyzed by ROC, and the AUC value was calculated. The results showed: 115 differential molecules with AUC≥0.700, among which 53 differential molecules with AUC≥0.750 (see Table 19). Among them, there are 9 differential molecules with AUC≥0.80, which are PS(39:4)-H, SM(d45:6)+H, PC(14:0/20:4)+HCOO, SM(d20:0/18:1)+HCOO, PS(49:4)-H, LPC(26:0)+HCOO, SM(d20:0/16:0)+HCOO, PA(50:4)-H, LPC(20:4)+HCOO.

**Table 19:** There are significant differences between the lipid molecular indexes of children with infantile hemangioma and those of normal children, and the lipid molecules with AUC≥0.75.

| Clas sific atio n | lipid molecule | average value_ control | Sd_ control | Avera ge IH | Sd_ THEM | multiple of differen ce | P-value | AUC |
|---|---|---|---|---|---|---|---|---|
| PS | PS(39:4)-H | 1.711 | 0.440 | 1.075 | 0.371 | 0.628 | <0.001 | 0.878 |
| SM | SM(d45:6) H | 0.038 | 0.011 | 0.022 | 0.013 | 0.587 | <0.001 | 0.834 |
| PC | PC(14:0/20: 4) HCOO | 0.183 | 0.050 | 0.111 | 0.059 | 0.603 | <0.001 | 0.833 |
| SM | SM(d20:0/18: 1) HCOO | 0.413 | 0.155 | 0.235 | 0.116 | 0.570 | <0.001 | 0.831 |
| PS | PS(49:4)-H | 0.076 | 0.021 | 0.047 | 0.025 | 0.617 | <0.001 | 0.823 |
| LPC | LPC(26:0) HCOO | 0.062 | 0.033 | 0.027 | 0.017 | 0.435 | <0.001 | 0.820 |
| SM | SM(d20:0/16: 0) HCOO | 6.307 | 2.062 | 3.844 | 1.858 | 0.609 | <0.001 | 0.810 |
| Well | PA(50:4)-H | 3.703 | 1.791 | 1.845 | 1.451 | 0.498 | <0.001 | 0.809 |
| LPC | LPC(20:4) HCOO | 6.483 | 1.589 | 4.074 | 2.231 | 0.628 | <0.001 | 0.800 |
| PC | PC(36:5) H | 0.347 | 0.100 | 0.229 | 0.105 | 0.660 | <0.001 | 0.795 |
| LPC | LPC(20:1) HCOO | 0.054 | 0.018 | 0.035 | 0.016 | 0.639 | <0.001 | 0.787 |
| PC | PC(38:8) H | 0.049 | 0.016 | 0.031 | 0.018 | 0.623 | <0.001 | 0.783 |
| PC | PC(46:5) H | 0.013 | 0.004 | 0.008 | 0.005 | 0.635 | <0.001 | 0.783 |
| ON | FRI (18:0p/ 22:5)-H | 0.068 | 0.021 | 0.045 | 0.021 | 0.661 | <0.001 | 0.781 |
| SM | SM(d22:0/16: 0) HCOO | 3.249 | 1.211 | 1.946 | 1.190 | 0.599 | <0.001 | 0.780 |
| PC | PC(15:0/20: 4) HCOO | 0.257 | 0.074 | 0.162 | 0.097 | 0.629 | <0.001 | 0.779 |
| SM | SM(d18:0/20: 4) HCOO | 0.032 | 0.015 | 0.017 | 0.014 | 0.538 | <0.001 | 0.778 |
| LPC | LPC(22:4) HCOO | 0.084 | 0.024 | 0.054 | 0.031 | 0.642 | <0.001 | 0.778 |
| PI | PI(18:0/22: 4)-H | 0.114 | 0.035 | 0.075 | 0.038 | 0.655 | <0.001 | 0.777 |
| PC | PC(16:1/18: 2) HCOO | 0.988 | 0.360 | 0.613 | 0.386 | 0.621 | <0.001 | 0.776 |
| PS | PS(40:4)-H | 0.288 | 0.136 | 0.168 | 0.091 | 0.583 | <0.001 | 0.773 |
| Cer | Cer(d20:0/24: 1) H | 0.007 | 0.003 | 0.004 | 0.002 | 0.590 | <0.001 | 0.771 |
| SM | SM(d37:0) HCOO | 0.132 | 0.057 | 0.077 | 0.049 | 0.586 | <0.001 | 0.771 |
| PC | PC(17:1/16: 0) HCOO | 0.780 | 0.241 | 0.521 | 0.245 | 0.668 | <0.001 | 0.769 |
| SM | SM(d20:0/22: 5) HCOO | 0.575 | 0.200 | 0.344 | 0.240 | 0.599 | <0.001 | 0.769 |
| PC | PC(44:11) H | 0.007 | 0.003 | 0.004 | 0.003 | 0.578 | <0.001 | 0.767 |
| SM | SM(d32:0) HCOO | 0.348 | 0.128 | 0.215 | 0.139 | 0.617 | <0.001 | 0.766 |
| LPC | LPC(22:1) H | 0.006 | 0.004 | 0.004 | 0.003 | 0.604 | 0.003 | 0.766 |

(continued)

| Clas sific atio n | lipid molecule | average value_ control | Sd_ control | Avera ge IH | Sd_ THEM | multiple of differen ce | P-value | AUC |
|---|---|---|---|---|---|---|---|---|
| PC | PC(33:0e) H | 0.017 | 0.007 | 0.010 | 0.009 | 0.595 | <0.001 | 0.766 |
| SM | SM(d56:2 pO) H | 0.011 | 0.004 | 0.006 | 0.004 | 0.613 | <0.001 | 0.765 |
| SM | SM(d42:1) HCOO | 7.401 | 3.306 | 4.429 | 2.679 | 0.598 | <0.001 | 0.765 |
| LPC | LPC(17:1) HCOO | 0.062 | 0.023 | 0.040 | 0.020 | 0.651 | <0.001 | 0.763 |
| SM | SM(d17:0/18: 2) HCOO | 0.153 | 0.052 | 0.092 | 0.063 | 0.599 | <0.001 | 0.763 |
| Cer | Cer(d18:1/24: 0) H | 0.093 | 0.040 | 0.057 | 0.032 | 0.616 | <0.001 | 0.761 |
| SM | SM(d36:3) HCOO | 0.166 | 0.060 | 0.100 | 0.072 | 0.604 | <0.001 | 0.760 |
| AcC a | AcCa(14:2) H | 0.020 | 0.008 | 0.012 | 0.008 | 0.611 | <0.001 | 0.759 |
| Cer | Cer(d18:1/24: 0 O) H | 0.002 | 0.001 | 0.001 | 0.001 | 0.634 | <0.001 | 0.759 |
| ON | PE(20:0p/20: 4)-H | 0.123 | 0.033 | 0.082 | 0.050 | 0.664 | <0.001 | 0.759 |
| LPC | LPC(26:1) HCOO | 0.018 | 0.005 | 0.012 | 0.008 | 0.634 | <0.001 | 0.757 |
| PC | PC(37:5) H | 0.089 | 0.037 | 0.058 | 0.032 | 0.651 | 0.001 | 0.757 |
| SM | SM(d44:1) HCOO | 0.072 | 0.039 | 0.041 | 0.028 | 0.565 | <0.001 | 0.757 |
| Cer | Cer(d18: 1/26: 1) H | 0.005 | 0.002 | 0.003 | 0.002 | 0.659 | <0.001 | 0.756 |
| ON | PE(40:6e) H | 0.124 | 0.091 | 0.069 | 0.050 | 0.558 | 0.007 | 0.754 |
| TG | TG(18:1/20: 2/22:4) NH4 | 0.224 | 0.105 | 0.136 | 0.097 | 0.607 | 0.001 | 0.753 |
| SM | SM(d34:3) H | 0.041 | 0.018 | 0.026 | 0.015 | 0.627 | <0.001 | 0.752 |
| ON | PE(40:4)-H | 0.254 | 0.059 | 0.169 | 0.095 | 0.664 | <0.001 | 0.752 |
| SM | SM(d22:0/18: 2) HCOO | 0.560 | 0.225 | 0.357 | 0.211 | 0.638 | <0.001 | 0.752 |
| Cer | Cer(d18:0/22: 0) H | 0.074 | 0.029 | 0.048 | 0.023 | 0.659 | <0.001 | 0.752 |
| SM | SM(d44:0) HCOO | 0.028 | 0.012 | 0.019 | 0.013 | 0.669 | 0.006 | 0.751 |
| PI | PI(16:1/20: 4)-H | 0.013 | 0.004 | 0.008 | 0.005 | 0.669 | 0.001 | 0.751 |
| PC | PC(20:3/20: 4) HCOO | 0.297 | 0.107 | 0.191 | 0.107 | 0.643 | <0.001 | 0.750 |
| ON | PE(38:lp)-H | 0.010 | 0.003 | 0.006 | 0.005 | 0.637 | <0.001 | 0.750 |
| SM | SM(d39:0) HCOO | 0.211 | 0.087 | 0.131 | 0.079 | 0.617 | <0.001 | 0.750 |

**Fitting model verification example**

Substitute the metabolite content detected in the first sample of the positive group and the control group into the above proteomics model, metabolomics model, and fatty acid omics model to obtain the Y value and predict the result. The results showed that among the four models, the Y values of the specimens in the IH group and the control group were significantly different, indicating that the four models all had good predictive effects. (See Table 20 below for details)

**Table 20**

| Model | specimen | Y value | forecast result |
|---|---|---|---|
| swissport proteomics - predictive models | IH Sample-1 | 3.656 | Positive |
| | Control sample-1 | -2.855 | Negative |
| uniport proteomics - prediction model | IH Sample-1 | 6.371 | Positive |
| | Control sample-1 | -0.986 | Negative |
| Metabolomics - Predictive Modeling | IH Sample-1 | 0.191 | Positive |
| | Control sample-1 | -2.809 | Negative |
| Medium and Long Chain Fatty Acids - Prediction Model | IH Sample-1 | 23238.923 | Positive |
| | Control sample-1 | -119.474 | Negative |

**Verification example:**

Examples 1-6

This example is used to verify that the significantly different fatty acid index obtained in the present invention can distinguish infantile hemangioma patients, normal children and neonatal erythema patients. The situation of specific embodiments 1-6 is shown in the following table 21.

**Table 21**

| Example | Corresponding detection object | diseased part |
|---|---|---|
| Example 1 | Infantile hemangioma patient 01 | elbow fossa |
| Example 2 | Infantile hemangioma patient 02 | the back |
| Example 3 | Neonatal erythema patient 01 | head |
| Example 4 | Neonatal erythema patient 02 | head |
| Example 5 | Normal Child 01 | none |
| Example 6 | Normal Child 02 | none |

The patients of Examples 1-6 or normal children or patients with neonatal erythema were all tested for relevant indicators of the present invention on their umbilical cord blood samples according to the method described above after birth, and the following table 22 shows the specific test results.

**Table 22** Fatty acid detection results of umbilical cord blood samples

| fatty acid | Example 1 (IH child 01) | Example 3 (Child with neonatal erythema 01) | Example 5 (normal child 01) | Example 2 (IH child 02) | Example 4 (children with neonatal erythema 02) | Example 6 (normal child 02) | IH compared with normal children | IH compared with neonata l erythem a |
|---|---|---|---|---|---|---|---|---|
| C10:0 | 0.028729 | 0.017742 | 0.005644 | 0.050895 | 0.01759 | 0.0115453 | high | high |
| C11:0 | 0.028122 | 0.010517 | 0.011587 | 0.042064 | 0.022055 | 0.0146453 | high | high |
| C12:0 | 0.257488 | 0.138076 | 0.055995 | 0.28728 | 0.173222 | 0.1204446 | high | high |
| C14:0 | 4.351606 | 2.855441 | 1.376344 | 3.941233 | 2.873807 | 1.5072576 | high | high |
| C15:0 | 0.879939 | 0.770499 | 0.272857 | 1.137546 | 0.637028 | 0.2611202 | high | high |

(continued)

| fatty acid | Example 1 (IH child 01) | Example 3 (Child with neonatal erythema 01) | Example 5 (normal child 01) | Example 2 (IH child 02) | Example 4 (children with neonatal erythema 02) | Example 6 (normal child 02) | IH compared with normal children | IH compared with neonatal erythema |
|---|---|---|---|---|---|---|---|---|
| C15:1N5 | 40.81086 | 27.47554 | 8.071184 | 35.76797 | 28.15836 | 19.382075 | high | high |
| C16:0 | 161.7352 | 100.2018 | 52.09138 | 175.4939 | 122.9309 | 56.778036 | high | high |
| C17:0 | 1.407512 | 0.720874 | 0.3251 | 1.048992 | 0.701315 | 0.3122798 | high | high |
| C18:0 | 84.13666 | 44.8526 | 26.16085 | 105.8078 | 60.0022 | 25.229636 | high | high |
| C18:2N6 | 427.1287 | 80.70468 | 80.06693 | 98.618 | 72.02917 | 69.109764 | high | high |
| C20:0 | 6.136846 | 0.280865 | 0.141123 | 1.110242 | 0.433811 | 0.1408695 | high | high |
| C20:3N3 | 0.463305 | 0.651841 | 0.235134 | 0.342892 | 0.59305 | 0.1529013 | high | Low |
| C20:5N3 | 2.780144 | 0.286709 | 0.071016 | 1.111225 | 0.338828 | 0.08811 | high | high |
| C21:0 | 0.333794 | 0.073656 | 0.034127 | 0.127887 | 0.11605 | 0.0527508 | high | high |
| C22:1N9 | 22.57718 | 6.348533 | 3.175789 | 28.52626 | 11.03295 | 2.7116273 | high | high |
| C23:0 | 0.665975 | 0.057325 | 0.023877 | 0.334212 | 0.167604 | 0.0305484 | high | high |
| C24:0 | 3.583151 | 0.32263 | 0.097446 | 1.345779 | 0.544029 | 0.1220033 | high | high |
| C8:0 | 0.593575 | 0.042803 | 0.022378 | 0.107887 | 0.084731 | 0.0217722 | high | high |
| Total MUFA | 349.2461 | 143.8864 | 64.7684 | 161.6838 | 137.6948 | 69.29005 | high | high |
| Total SFA | 267.7383 | 156.2766 | 83.08047 | 292.8814 | 191.4751 | 86.04428 | high | high |

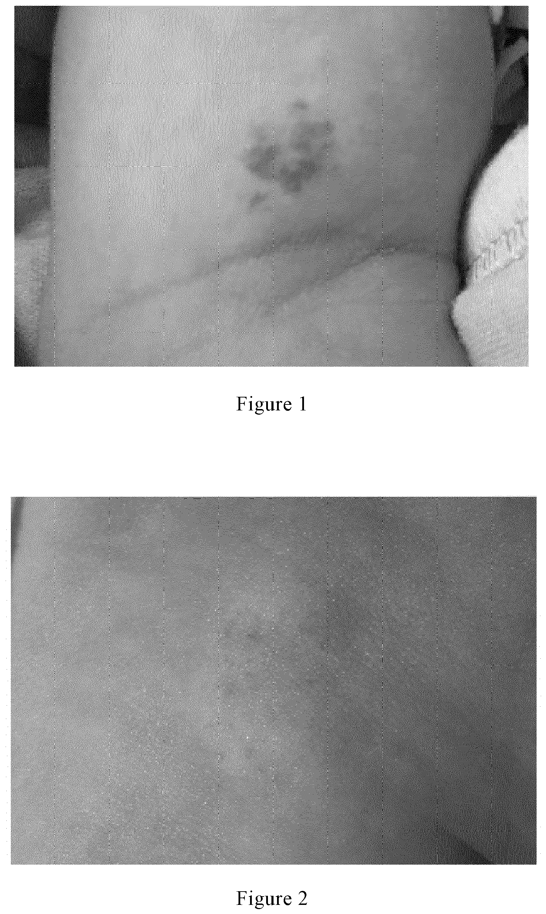

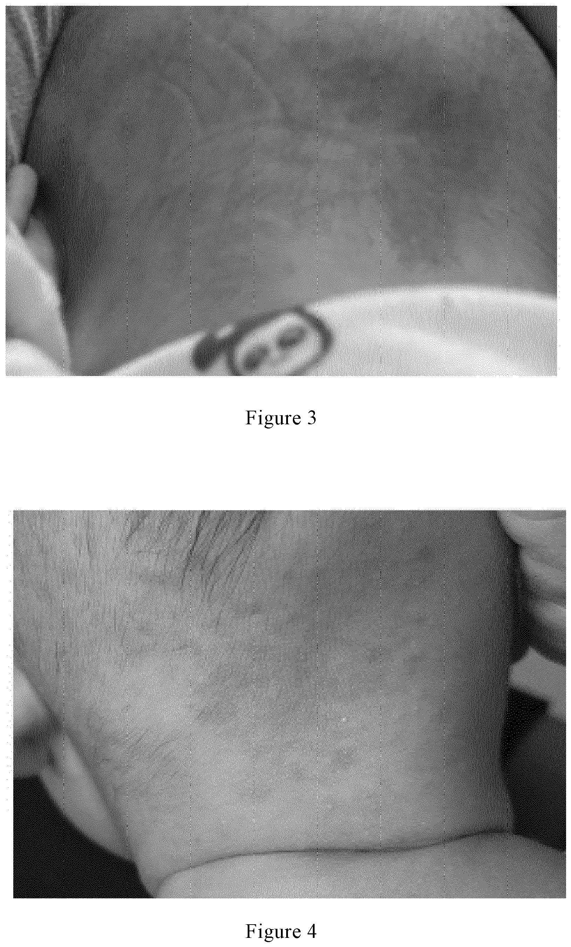

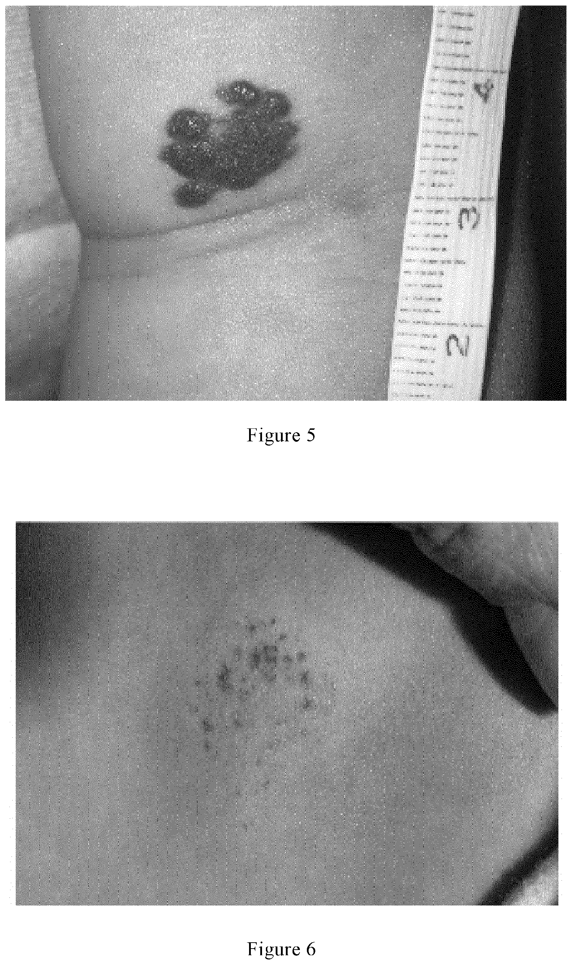

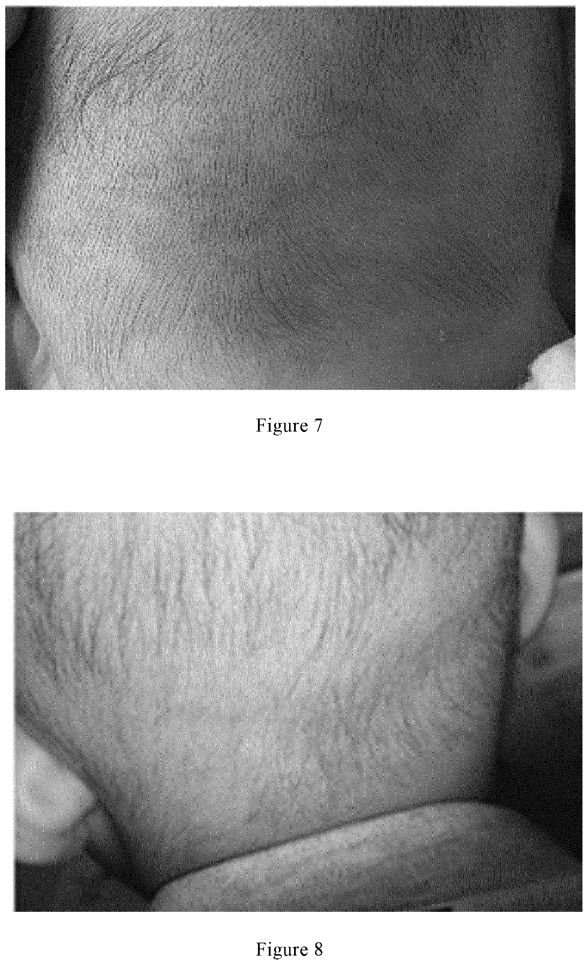

Figures 1 to 8 show the growth of lesions in patients with infantile hemangioma and in patients with neonatal erythema. Figures 1 and 2 show two infantile hemangioma patients with reddish red patches in the cubital fossa and back, respectively, at 6 weeks after birth, which are consistent with the symptoms of neonatal erythema patients in Figures 3 and 4 being not easy to distinguish; Figure 3 and Figure 4 show two patients with neonatal erythema at 6 weeks after birth, and occipital neonatal erythema also showed reddish red patches; Figure 5 and Figure 6 show two infants with blood vessels, at 12 weeks after birth, the infantile hemangiomas in the cubital fossa and back of the patient with hemangioma gradually increased in size and became darker in color. At 12 weeks after birth, the occipital neonatal erythema remained unchanged or faded in color. (Note: Parents of infantile hemangioma patients who agreed to participate in the experiment did not agree to start treatment for IH in advance for children whose umbilical cord blood samples showed abnormalities at the stage of no obvious onset before 12 weeks after birth.)

As can be seen from Table 22, detecting those indicators discovered by the inventors from their umbilical cord blood samples can screen out the accurate diagnosis of infantile hemangioma patients in advance, so as to timely control the disease incidence and development of infantile hemangioma patients offers possibilities.

Example 7-37

This example is used to verify that the significantly different metabolites, proteins, lipid subclasses and lipid molecular indicators obtained in the present invention can distinguish infantile hemangioma patients from normal children. The specific test results are shown in Table 23-26.

**Table 23** Metabolite Detection Results of Cord Blood Samples in Examples 7-16

| Metabolites | Example 7 (IH child 03) | Example 8 (normal child 03) | IH compared with normal children |
|---|---|---|---|
| (3-carboxypropyl) trimethylammoniu m cation | 510143.1 | 227093.9 | high |
| α-D-( )-talose | 23263.73 | 15500.56 | high |
| 1-Aminocyclopropanecarboxylic acid | 188621.6 | 53239.43 | high |
| 1-myristoyl-sn-glycero-3-phosphocholi ne | 639233.5 | 186504.9 | high |
| 1-palmitoyl-sn-glycero-3-phosphocholi ne | 28013391 | 5866421 | high |
| 1-Stearoyl-sn-glycero-3-phosphocholin e | 373926.7 | 113154.4 | high |
| choline | 227648.3 | 91692.5 | high |
| DL-indole-3-lactic acid | 1300046 | 461850.9 | high |
| Glycerophosphorylcholine | 1688828 | 671972.8 | high |
| L-Arginine | 5502806 | 1172718 | high |
| L-glutamic acid | 240640.1 | 33478.38 | high |
| L-phenylalanine | 785222.5 | 311896.4 | high |
| L-tryptophan | 258068.4 | 88296.11 | high |
| NG,NGDimethyl-L-arginine | 2772705 | 953256.3 | high |
| Tyramine | 1792274 | 781770.9 | high |
| (S)-Citromalic acid | 33227.41 | 4088.282 | high |
| 1-Oleoyl-L-α-lysophosphatidic acid | 639392.5 | 63903.35 | high |
| 1-palmitoyl lysophosphatidic acid | 124968.9 | 4535.698 | high |
| α-mangostin | 670186.4 | 310314.6 | high |
| Arachidonic Acid (peroxide free) | 1260791 | 284498.3 | high |
| citric acid | 94447.07 | 1097.373 | high |
| cyclamate | 24786.94 | 16467.89 | high |
| D(-)-beta-Hydroxybutyrate | 69074.85 | 34237.41 | high |
| Inositol galactoside | 2440690 | 948031 | high |
| glyceric acid | 137497.5 | 31005.86 | high |
| L-Aspartic Acid | 143217.9 | 21282.67 | high |
| L-Carnosine | 4436.155 | 71437.44 | Low |
| L-malic acid | 176454.5 | 5539.126 | high |
| N-acetyl-L-aspartic acid | 69885.77 | 22113.51 | high |
| N-acetylneuraminic acid | 128129.9 | 16034.11 | high |

(continued)

| Metabolites | Example 7 (IH child 03) | Example 8 (normal child 03) | IH compared with normal children |
|---|---|---|---|
| norethindrone acetate | 223347.9 | 51205.17 | high |
| Phosphocholine | 89380.63 | 19148.07 | high |
| sn-glycero-3-phosphoethanolamine | 369254.4 | 48702.83 | high |
| Succinate | 101197.9 | 18696.45 | high |

**Continued Table 23**

| Metabolites | Example 9 (IH child 04) | Example 10 (normal child 04) | IH compared with normal children |
|---|---|---|---|
| (3-carboxypropyl) trimethylammoniu m cation | 320168.9 | 232712.5 | high |
| $\alpha$-D-( )-talose | 53267.22 | 7886.056 | high |
| 1-Aminocyclopropanecarboxylic acid | 97254.68 | 69077.64 | high |
| 1-myristoyl-sn-glycero-3-phosphocholi ne | 345230.1 | 60107.51 | high |
| 1-oleoyl-sn-glycero-3-phosphocholine | 225774.6 | 41495.52 | high |
| 1-Stearoyl-sn-glycero-3-phosphocholi ne | 558217.3 | 109993.2 | high |
| Bilirubin | 850090.4 | 60025.95 | high |
| choline | 205465.2 | 138419.4 | high |
| D-2-pipericolic acid | 454456.9 | 258992.6 | high |
| DL-indole-3-lactic acid | 868161.1 | 200081.4 | high |
| Glycyl glutamate | 53138.69 | 19522.78 | high |
| Glycerophosphorylcholine | 1915716 | 865545.9 | high |
| Isomaltose | 608459.3 | 72821.55 | high |
| L-glutamic acid | 152540.5 | 107752 | high |
| L-tryptophan | 180972.2 | 40983.08 | high |
| NG,NGDimethyl-L-arginine | 1639060 | 752907.1 | high |
| Phenylacetic acid | 22581.97 | 30875.42 | Low |
| (S)-Citromalic acid | 70528.46 | 2136.355 | high |
| 1-Oleoyl-L-$\alpha$-lysophosphatidic acid | 136196.3 | 22149.72 | high |
| 1-palmitoyl lysophosphatidic acid | 20645.84 | 4453.156 | high |
| citric acid | 43327.91 | 1825.697 | high |
| D-threitol | 884446.3 | 103468 | high |

(continued)

| Continued Table 23 | | | |
|---|---|---|---|
| Metabolites | Example 9 (IH child 04) | Example 10 (normal child 04) | IH compared with normal children |
| Inositol galactoside | 4131153 | 482120 | high |
| glyceric acid | 66956.89 | 49771.75 | high |
| L-Aspartic Acid | 75412.97 | 28727.6 | high |
| L-Carnosine | 27469.61 | 59068.78 | Low |
| L-malic acid | 73974.38 | 8678.877 | high |
| N-acetyl-L-aspartic acid | 38754.87 | 20086.51 | high |
| N-acetylneuraminic acid | 86250.82 | 19123.33 | high |
| norethindrone acetate | 161323.2 | 55845.14 | high |
| Phosphocholine | 61866.2 | 28427.71 | high |
| sn-glycero-3-phosphoethanolamine | 202229 | 102070.7 | high |
| Succinate | 60314.99 | 20776.69 | high |
| Continued Table 23 | | | |
| | eao a | ) e | )IH |
| (3-carboxypropyl)tr | | | |
| α | | | |
| 1-Aminocyclopropanecar | | | |
| 1-Stearoyl-sn-glycero-3-p | | | |
| | | | |
| | | | |
| -pipericoli | | | |
| glutam | | | |
| phorylcholne16 | | | |
| | | | |
| 115 | | | |
| 24294 | | al | |
| h | | | |
| Ta | | | |
| | | | |
| pspa | | | |
| 442840h | 95 | . | |
| | | h | |
| | - | | |
| | | | |
| eri | d | 5 | |

| Continued Table 23 | | | |
|---|---|---|---|
| | eao a | ) e | )IH |
| i17 | | | |
| ia | 6 | | |
| | | | |
| | | | |
| | | | |
| | | | |
| N-acetyl-L-aspartic acid | 51911.12 | 14963.55 | high |
| N-acetylneuraminic acid | 118212.1 | 22109.31 | high |
| norethindrone acetate | 209976.5 | 111536.7 | high |
| Phosphocholine | 46142.03 | 15933.69 | high |
| sn-glycero-3-phosphoethanolamine | 252622.7 | 38283.66 | high |
| Succinate | 86850.85 | 17127.58 | high |

| Continued Table 23 | | | |
|---|---|---|---|
| Metabolites | Example 13 (IH child 06) | Example 14 (normal child 06) | IH compared with normal children |
| (3-carboxypropyl)trimethylammoniu m cation | 629036.2 | 230257.3 | high |
| $\alpha$-D-( )-talose | 44115.07 | 16960.69 | high |
| 1-Aminocyclopropanecarboxylic acid | 94329.05 | 49168.4 | high |
| 1-myristoyl-sn-glycero-3-phosphochol ine | 580831.1 | 179074.7 | high |
| 1-oleoyl-sn-glycero-3-phosphocholine | 166464.6 | 59803.85 | high |
| 1-palmitoyl-sn-glycero-3-phosphochol ine | 31923571 | 5171830 | high |
| choline | 307412.2 | 108971.7 | high |
| DL-indole-3-lactic acid | 1837840 | 521566.6 | high |
| Glycerophosphorylcholine | 1355195 | 692020.8 | high |
| Isomaltose | 414269 | 152420.9 | high |
| L-glutamic acid | 140465.1 | 23508.76 | high |
| L-phenylalanine | 996992.2 | 401872.1 | high |
| L-tryptophan | 360420.5 | 103943 | high |
| Tyramine | 2133112 | 973692.5 | high |
| Phenylacetic acid | 20765.4 | 31707.5 | Low |
| (S)-Citromalic acid | 12353.27 | 4792.731 | high |
| $\alpha$-mangostin | 916127.6 | 231627.4 | high |
| cyclamate | 48629.76 | 15112.53 | high |
| D-galactate | 31600.3 | 55556.12 | Low |
| Inositol galactoside | 3007417 | 982875.2 | high |
| glyceric acid | 79903.29 | 27278.19 | high |

(continued)

| Continued Table 23 | | | |
|---|---|---|---|
| Metabolites | Example 13 (IH child 06) | Example 14 (normal child 06) | IH compared with normal children |
| L-Aspartic Acid | 102114.2 | 11808.49 | high |
| L-Carnosine | 38562.33 | 112370.8 | Low |
| L-malic acid | 102599.6 | 6392.867 | high |
| N-acetyl-L-aspartic acid | 38679.11 | 13727.85 | high |
| N-acetylneuraminic acid | 176493.9 | 22262.77 | high |
| Phosphocholine | 58819.83 | 20118.58 | high |
| sn-glycero-3-phosphoethanolamine | 339402.5 | 50557.39 | high |
| Succinate | 58534.13 | 13912.63 | high |

| Continued Table 23 | | |
|---|---|---|
| Metabolites | Example 15 (IH child 07) | Example 16 (normal child 07) | IH compared with normal children |
| $\alpha$-D-( )-talose | 21646.92 | 13619.36 | high |
| 1-Aminocyclopropanecarboxylic acid | 128914.9 | 53073.99 | high |
| choline | 203330 | 97026.51 | high |
| D-2-pipericolic acid | 467372 | 325019.8 | high |
| Glycyl glutamate | 36759.24 | 7489.737 | high |
| Glycerophosphorylcholine | 1885700 | 653829.6 | high |
| L-glutamic acid | 224648.1 | 24824.15 | high |
| NG,NG-Dimethyl-L-arginine | 1475795 | 559897.5 | high |
| (S)-Citromalic acid | 16108.88 | 5697.663 | high |
| 1-Oleoyl-L-$\alpha$-lysophosphatidic acid | 139287.4 | 26931.92 | high |
| 1-palmitoyl lysophosphatidic acid | 15066.68 | 3648.164 | high |
| Arachidonic Acid (peroxide free) | 428874.9 | 290231.9 | high |
| citric acid | 32548.69 | 1109.416 | high |
| Inositol galactoside | 1459116 | 865594.7 | high |
| glyceric acid | 93033.19 | 46310.2 | high |
| L-Aspartic Acid | 101596.3 | 19824.56 | high |
| L-Carnosine | 15087.77 | 65887.83 | Low |
| L-malic acid | 41259.35 | 11124.48 | high |
| N-acetyl-L-aspartic acid | 44439.19 | 15711.84 | high |
| N-acetylneuraminic acid | 71809.25 | 27744.24 | high |
| Phosphocholine | 62950 | 20583.36 | high |

(continued)

| Continued Table 23 | | | |
|---|---|---|---|
| Metabolites | Example 15 (IH child 07) | Example 16 (normal child 07) | IH compared with normal children |
| sn-glycero-3-phosphoethanolami ne | 238898.5 | 55692.48 | high |
| Succinate | 45201.87 | 17689.08 | high |

**Table 24** The protein detection results of the umbilical cord blood samples of Examples 17-26

| protein | Example 17 (IH child 08) | Example 18 (IH child 08) | IH compared with normal children |
|---|---|---|---|
| COLLECTION10 | 489637.08 | 37176.48 | high |
| CPQ | 50232.41 | 6951.64 | high |
| LDHB | 80543.82 | 37105.88 | high |
| not | 345978.53 | 34287.12 | high |
| CPN1 | 174232.73 | 315119.13 | Low |
| PROC | 86069.12 | 140235.3 | Low |
| SUPT6H | 27748.59 | 10059.42 | high |
| BCAR3 | 497794 | 318584.44 | high |
| SOX30 | 122983.34 | 250598.77 | Low |
| MON1 | 884588.38 | 1645950.4 | Low |
| IGKV1D-16 | 403878.31 | 244457.31 | high |
| COMP | 56597.46 | 34517.18 | high |
| FUCA2 | 31612.46 | 121781.38 | Low |
| HLA-B | 11852718 | 8710737 | high |
| FKBP1A | 26359.98 | 52192.52 | Low |
| SAA1 | 17142.71 | 103461.22 | Low |
| PGD | 153134.02 | 72877.83 | high |
| IGKV6-21 | 345471.18 | 243696.84 | high |
| C1QA | 1679422 | 1406025.3 | high |
| APP | 27362.17 | 22903.19 | high |

| Continued Table 24 | | | |
|---|---|---|---|
| protein | Example 19 (IH child 09) | Example 20 (IH child 09) | IH compared with normal children |
| COLLECTION10 | 1694103.5 | 30962.67 | high |
| not | 2405567.3 | 22159.97 | high |
| KRT1 | 1201667.6 | 338370.22 | high |
| POSTN | 38748.93 | 92234.64 | Low |
| LAMA5 | 91258.05 | 325520.4 | Low |

(continued)

|  | Continued Table 24 | | |
| protein | Example 19 (IH child 09) | Example 20 (IH child 09) | IH compared with normal children |
|---|---|---|---|
| CPN1 | 176122.81 | 312381.63 | Low |
| PROC | 59420.95 | 167681.98 | Low |
| BCAR3 | 453915.63 | 322910.13 | high |
| SOX30 | 46228.37 | 212304.75 | Low |
| PROC | 50152.57 | 159895.33 | Low |
| IGKV1D-16 | 423852.69 | 223151.53 | high |
| PMFBP1 | 36022 | 57853.33 | Low |
| FGFBP2 | 212745.72 | 26039.64 | high |
| HSPE1 | 919539 | 7279872.4 | Low |
| PGD | 574242.25 | 92360.25 | high |
| SOD1 | 66378.97 | 48647.85 | high |
| COTL1 | 36463.08 | 108060.94 | Low |
| CAT | 66668.719 | 1332205.3 | Low |
| PROS1 | 232601.3 | 191883.78 | high |
| CD5L | 288586.03 | 124116.71 | high |
| HMFT1766 | 703811.06 | 574892.5 | high |

|  | Continued Table 24 | | |
| protein | Example 21 (Children with IH 10) | Example 22 (Children with IH 10) | IH compared with normal children |
|---|---|---|---|
| not | 2584658.5 | 41713.85 | high |
| KRT1 | 2446204.3 | 166468.95 | high |
| ARHGDIB | 12051.3 | 872110.06 | Low |
| PROC | 75541.1 | 141050.69 | Low |
| C1S | 849727.19 | 1133271.1 | Low |
| SUPT6H | 29087.84 | 15165.92 | high |
| bche | 139511.2 | 335381.53 | Low |
| C1R | 269981.13 | 395077.63 | Low |
| PROC | 51921.99 | 105892.16 | Low |
| IGKV1D-16 | 390209.81 | 248376.56 | high |
| COMP | 64995.19 | 41509.69 | high |
| HSPE1 | 35990.05 | 15256183 | Low |
| CDH1 | 33020.29 | 57182.13 | Low |
| ISLR | 11837.14 | 34257.99 | Low |
| CORO1A | 50342.75 | 81949.86 | Low |
| ZYX | 65370.4 | 108204.31 | Low |

(continued)

| Continued Table 24 | | | |
|---|---|---|---|
| protein | Example 21 (Children with IH 10) | Example 22 (Children with IH 10) | IH compared with normal children |
| A0A1W6IYJ 2 | 153517.8 | 2168239.5 | Low |
| PRDX1 | 75655.39 | 284166.06 | Low |
| CAT | 41240.133 | 743673.63 | Low |
| PROS1 | 284338.66 | 197126.7 | high |
| CD5L | 248540.08 | 146032.84 | high |
| HMFT1766 | 665014.5 | 493370.72 | high |
| KRT2 | 317589 | 32842.34 | high |

| Continued Table 24 | | | |
|---|---|---|---|
| protein | Example 23 (Children with IH 11) | Example 24 (Children with IH 11) | IH compared with normal children |
| COLLECTION 10 | 3388055.3 | 40016.11 | high |
| POSTN | 41532.77 | 92365.08 | Low |
| PROC | 56539.59 | 149606.16 | Low |
| C1S | 742738.81 | 966209.13 | Low |
| SUPT6H | 32310.08 | 10553.28 | high |
| BCAR3 | 502538.63 | 216837.97 | high |
| bche | 166995.64 | 277742.38 | Low |
| SOX30 | 121270.22 | 233738.78 | Low |
| C1R | 289904.19 | 359219.97 | Low |
| IGKV1D-16 | 425436.22 | 264957.34 | high |
| APOC3 | 3651022.5 | 5632990.5 | Low |
| GSN | 505719.47 | 866859.94 | Low |
| SELL | 121987.74 | 159236.16 | Low |
| CDH1 | 24045.53 | 90848.18 | Low |
| ZYX | 30331.08 | 128526.67 | Low |
| SORL1 | 5664647.5 | 971401.38 | high |
| FAM3C | 13536.82 | 18625.73 | Low |
| CST6 | 22128.1 | 88522.59 | Low |
| PRDX1 | 186841.38 | 559074.75 | Low |
| C1QA | 1726583 | 1364217.3 | high |
| APP | 45928.97 | 21351.4 | high |

| Continued Table 24 | | | |
|---|---|---|---|
| protein | Example 25 (12 children with IH) | Example 26 (12 children with IH) | IH compared with normal children |
| KRT1 | 457461.5 | 223820.53 | high |

(continued)

| Continued Table 24 | | | |
|---|---|---|---|
| protein | Example 25 (12 children with IH) | Example 26 (12 children with IH) | IH compared with normal children |
| LAMAS | 93517.72 | 1558859.5 | Low |
| HPX | 1283580.4 | 2786933.8 | Low |
| CFHR4 | 312037.53 | 48870.82 | high |
| PROC | 45383.84 | 90516.25 | Low |
| IGKV1D-1 6 | 384415.78 | 55965.74 | high |
| SELENOP | 221991.19 | 147792.09 | high |
| PMFBP1 | 37402.39 | 46928.19 | Low |
| GSN | 726584.06 | 1053384.4 | Low |
| SELL | 106793.16 | 190474.09 | Low |
| HLA-B | 10611360 | 6585273.5 | high |
| FKBP1A | 19399.96 | 34309.7 | Low |
| PGD | 212678.84 | 31996.6 | high |
| SOD1 | 71198.32 | 42425.97 | high |
| ISLR | 19732.98 | 29068.05 | Low |
| RLBP1 | 475881.03 | 197928.81 | high |
| IDH1 | 55569.85 | 13489.66 | high |
| IGKV6-21 | 1081120.9 | 129328.91 | high |
| FAM3C | 6287.55 | 27064.79 | Low |
| CST6 | 36840.92 | 47662.6 | Low |
| COTL1 | 60569.28 | 113931.08 | Low |
| PIGR | 812006.8 | 209093.87 | high |
| HMFT1766 | 560376.88 | 462474.25 | high |
| KRT2 | 43344.047 | 16670.71 | high |

**Table 25** The detection results of lipid subclasses in the umbilical cord blood samples of Examples 27-32

| lipid subclass | Example 27 (13 children with IH) | Example 28 (normal children 13) | Example 29 (14 children with IH) | Example 30 (normal children 14) | Example 31 (Children with IH 15) | Example 32 (normal children 15) | IH compared with normal children |
|---|---|---|---|---|---|---|---|
| AcCa | 0.321158 | 1.064138 | 0.321049 | 0.896335 | 0.314356 | 0.918663 | Low |
| Cer | 0.351728 | 1.533944 | 0.546616 | 1.720177 | 0.379785 | 1.507481 | Low |
| CerG1 | 0.07868 | 0.381009 | 0.104414 | 0.370528 | 0.066404 | 0.361331 | Low |
| CerG2 | 0.040803 | 0.191394 | 0.05011 | 0.174356 | 0.051909 | 0.191599 | Low |
| CerG2GN Ac1 | 0.100713 | 0.455635 | 0.113086 | 0.444541 | 0.11744 | 0.465601 | Low |
| CerG3 | 0.004797 | 0.029168 | 0.005124 | 0.022737 | 0.005923 | 0.019632 | Low |
| That | 380.889 | 1477.079 | 353.2145 | 1357.131 | 378.9874 | 1299.011 | Low |

(continued)

| lipid subclass | Example 27 (13 children with IH) | Example 28 (normal children 13) | Example 29 (14 children with IH) | Example 30 (normal children 14) | Example 31 (Children with IH 15) | Example 32 (normal children 15) | IH compared with normal children |
|---|---|---|---|---|---|---|---|
| CL | 0.048121 | 0.115846 | 0.036232 | 0.087427 | 0.028412 | 0.114302 | Low |
| GM3 | 0.412922 | 0.764051 | 0.536102 | 0.8271 | 0.43535 | 0.839201 | Low |
| LPC | 30.01321 | 76.91753 | 33.96081 | 84.09475 | 31.96249 | 78.38407 | Low |
| LPE | 2.140922 | 5.229405 | 2.496672 | 5.956277 | 2.860132 | 5.781476 | Low |
| MGDG | 0.010424 | 0.03236 | 0.010329 | 0.032346 | 0.01034 | 0.033013 | Low |
| Well | 0.683589 | 3.869103 | 0.417211 | 6.172396 | 0.520085 | 4.368819 | Low |
| PC | 212.0522 | 629.5946 | 252.3592 | 685.9719 | 214.5748 | 589.463 | Low |
| ON | 6.947867 | 20.06681 | 6.812983 | 19.2329 | 6.519104 | 18.61558 | Low |
| phSM | 2.517173 | 1.669277 | 2.425129 | 1.918006 | 2.597779 | 2.024251 | high |
| PI | 12.00532 | 22.26124 | 13.68311 | 19.8602 | 12.94378 | 19.11935 | Low |
| PS | 489.2272 | 758.3536 | 480.6222 | 782.0371 | 553.1318 | 734.6101 | Low |
| SM | 78.72611 | 346.3847 | 71.48079 | 328.4313 | 80.3259 | 316.4796 | Low |
| TG | 107.5121 | 342.3344 | 117.0316 | 343.6731 | 57.17036 | 407.0348 | Low |

**Table 26** Lipid molecular detection results of umbilical cord blood samples in Examples 33-36

| lipid molecule | Example 33 (16 children with IH) | Example 34 (normal children 16) | Example 35 (17 children with IH) | Example 36 (normal children 17) | IH compared with normal children |
|---|---|---|---|---|---|
| PS(39:4)-H | 0.65898 | 2.773197 | 0.505236 | 1.562799 | Low |
| SM(d45:6) H | 0.008445 | 0.042886 | 0.003311 | 0.032579 | Low |
| PC(14:0/20:4) HCOO | 0.052594 | 0.30468 | 0.060199 | 0.216308 | Low |
| SM(d20:0/18:1) HCOO | 0.154861 | 0.475846 | 0.091844 | 0.605941 | Low |
| PS(49:4)-H | 0.010045 | 0.136636 | 0.006305 | 0.057244 | Low |
| LPC(26:0) HCOO | 0.006137 | 0.112732 | 0.008202 | 0.083598 | Low |
| SM(d20:0/16:0) HCOO | 2.373884 | 4.600755 | 2.028368 | 10.25497 | Low |
| PA(50:4)-H | 0.465445 | 2.706362 | 0.758617 | 8.060536 | Low |
| LPC(20:4) HCOO | 1.486188 | 8.882914 | 2.65547 | 6.922193 | Low |

(continued)

| lipid molecule | Example 33 (16 children with IH) | Example 34 (normal children 16) | Example 35 (17 children with IH) | Example 36 (normal children 17) | IH compared with normal children |
|---|---|---|---|---|---|
| PC(36:5) H | 0.135514 | 0.498213 | 0.180945 | 0.419179 | Low |
| LPC(20: 1) HCOO | 0.020232 | 0.054994 | 0.034078 | 0.069364 | Low |
| PC(38:8) H | 0.016202 | 0.073509 | 0.022128 | 0.062792 | Low |
| PC(46:5) H | 0.004694 | 0.021572 | 0.002089 | 0.014555 | Low |
| FRI (18: 0p/22: 5)-H | 0.025236 | 0.103705 | 0.044721 | 0.07165 | Low |
| SM(d22: 0/16:0) HCOO | 0.934436 | 2.625851 | 0.705129 | 5.44385 | Low |
| PC(15: 0/20:4) HCOO | 0.05268 | 0.433913 | 0.042367 | 0.33059 | Low |
| SM(d18: 0/20:4) HCOO | 0.002063 | 0.02705 | 0.00094 | 0.068781 | Low |
| LPC(22: 4) HCOO | 0.035659 | 0.150585 | 0.031885 | 0.082646 | Low |
| PI(18: 0/22:4)-H | 0.048502 | 0.15303 | 0.080136 | 0.127058 | Low |
| PC(16: 1/18:2) HCOO | 0.180617 | 1.099495 | 0.132978 | 1.078421 | Low |
| PS(40: 4)-H | 0.079248 | 0.523989 | 0.018216 | 0.26626 | Low |
| Cer(d20: 0/24:1) H | 0.002263 | 0.008674 | 0.001705 | 0.006754 | Low |
| SM(d37: 0) HCOO | 0.032263 | 0.131899 | 0.01664 | 0.215812 | Low |
| PC(17: 1/16:0) HCOO | 0.316828 | 1.431112 | 0.243384 | 0.90886 | Low |
| SM(d20: 0/22:5) HCOO | 0.107566 | 0.60368 | 0.16029 | 0.893717 | Low |
| PC(44: 11) H | 0.001781 | 0.007933 | 0.000836 | 0.008616 | Low |
| SM(d32: 0) HCOO | 0.074281 | 0.363346 | 0.083426 | 0.671339 | Low |

(continued)

| lipid molecule | Example 33 (16 children with IH) | Example 34 (normal children 16) | Example 35 (17 children with IH) | Example 36 (normal children 17) | IH compared with normal children |
|---|---|---|---|---|---|
| LPC(22: 1) H | 0.001862 | 0.007073 | 0.003268 | 0.006216 | Low |
| PC(33: 0e) H | 0.001795 | 0.025562 | 0.003189 | 0.021722 | Low |
| SM(d56: 2 pO) H | 0.003127 | 0.014044 | 0.004206 | 0.013686 | Low |
| SM(d42: 1) HCOO | 2.109844 | 9.187529 | 1.48469 | 13.52494 | Low |
| LPC(17: 1) HCOO | 0.016819 | 0.124613 | 0.027881 | 0.061764 | Low |
| SM(d17: 0/18:2) HCOO | 0.022482 | 0.205564 | 0.019586 | 0.240992 | Low |
| Cer(d18: 1/24:0) H | 0.034595 | 0.105274 | 0.041222 | 0.097188 | Low |
| SM(d36: 3) HCOO | 0.021995 | 0.209217 | 0.03407 | 0.234692 | Low |
| AcCa(14: 2) H | 0.009365 | 0.017994 | 0.013856 | 0.02766 | Low |
| Cer(d18: 1/24:0 O) H | 0.000219 | 0.002624 | 0.001069 | 0.002788 | Low |
| PE(20: 0p/20: 4)-H | 0.043925 | 0.117114 | 0.034398 | 0.135764 | Low |
| LPC(26: 1) HCOO | 0.00197 | 0.028354 | 0.002493 | 0.014764 | Low |
| PC(37:5) H | 0.018959 | 0.138687 | 0.017524 | 0.108174 | Low |
| SM(d44: 1) HCOO | 0.015268 | 0.093548 | 0.010586 | 0.124478 | Low |
| Cer(d18: 1/26:1) H | 0.002071 | 0.005326 | 0.002575 | 0.005759 | Low |
| PE(40: 6e) H | 0.052156 | 0.35594 | 0.037536 | 0.159764 | Low |
| TG(18: 1/20: 2/22:4) NH4 | 0.068634 | 0.269178 | 0.029737 | 0.202736 | Low |
| SM(d34: 3) H | 0.013771 | 0.051332 | 0.014828 | 0.063531 | Low |
| PE(40: 4)-H | 0.035489 | 0.245829 | 0.025996 | 0.254869 | Low |

(continued)

| lipid molecule | Example 33 (16 children with IH) | Example 34 (normal children 16) | Example 35 (17 children with IH) | Example 36 (normal children 17) | IH compared with normal children |
|---|---|---|---|---|---|
| SM(d22: 0/18:2) HCOO | 0.137212 | 0.421372 | 0.097646 | 1.111903 | Low |
| Cer(d18: 0/22:0) H | 0.042999 | 0.07261 | 0.041678 | 0.073546 | Low |
| SM(d44: 0) HCOO | 0.009909 | 0.026304 | 0.001866 | 0.032173 | Low |
| PI(16: 1/20:4)-H | 0.001363 | 0.01667 | 0.006786 | 0.011267 | Low |
| PC(20: 3/20:4) HCOO | 0.108377 | 0.278518 | 0.143706 | 0.503058 | Low |
| PE(38: 1p)-H | 0.000607 | 0.014607 | 0.001942 | 0.010799 | Low |
| SM(d39: 0) HCOO | 0.056764 | 0.245858 | 0.037246 | 0.356859 | Low |

### Example 37:

This example is used to illustrate the use of the indicators of the present invention for monitoring the progress of IH and the therapeutic effect of drugs.

As shown in Figure 9: the regression of the left upper eyelid infantile hemangioma after oral administration of propranolol. (A) Before starting to take propranolol, the tumor hyperplasia was obvious; (B) After taking propranolol for 1 month, the tumor gradually subsided; (C) After taking propranolol for 12 months, the IH basically disappeared and stopped (D) After 2 months of drug withdrawal, IH rebounded and grew.

**Table 27:** Changes of fatty acid C20:0 and C22:6N3 concentrations in peripheral blood before and after oral administration of propranolol in children with IH.

| treatment history | C20:0 ($\mu$g/ml) | C22:6N3 ($\mu$g/ml) |
|---|---|---|
| before starting treatment | 1.58 | 12.30 |
| Oral propranolol for 1 month | 0.67 | 9.71 |
| Oral propranolol for 12 months | 0.57 | 7.77 |
| Withdrawal for 2 months | 0.99 | 8.66 |

It can be seen from Table 27 that the fatty acid index of the present invention can effectively monitor the progress of IH and the therapeutic effect of drugs.

Although the subject matter of this disclosure and its merits have been described in detail, it should be understood that variations, substitutions and alterations may be made without deviating from the spirit and scope of the invention as defined by the attached claims. Furthermore, the scope of this application is not intended to be limited to a specific implementation of the uses, substance compositions, kits, methods and steps described in the specification. The general skilled person in the field will easily understand from the disclosure of the disclosed topic that a use, substance composition, kit, method, or step, existing or to be developed according to this disclosed topic, may be used that performs substantially the same function or achieves substantially the same results as the corresponding implementation described herein. Therefore, the attached claims are intended to include such uses, substance compositions, kits, methods or steps within their scope.

Patents, patent applications, publications, product descriptions and protocols are cited throughout this application, the disclosures of which are hereby incorporated by reference in their entirety for all purposes.

**Claims**

1. Use of a composition for manufacturing a medicament for diagnosing infantile hemangioma and/or monitoring its progress and prognosis is **characterized in that** the composition includes at least one of the following four groups of substances:

(1) Fatty acid, wherein the fatty acid is total fatty acid or total saturated fatty acid or total monounsaturated fatty acid or at least one of the fatty acids selected from the group consisting of C10:0, CII:0, C12:0, C14:0, C15:0, C15:1N5, C16:0, C17:0, C18:0, C18:2N6, C20:0, C20:3N3, C20:5N3, C21:0, C22:1N9, C23:0, C24:0 and C8:0;

(2) Metabolites, wherein the metabolites is at least one of the metabolites selected from the group consisting of (3-carboxypropyl)trimethylammonium cation, $\alpha$-D-(+)-talose, 1-aminocyclopropanecarboxylic acid, 1-meat Myristoyl-sn-glycero-3-phosphocholine, 1-oleoyl-sn-glycero-3-phosphocholine, 1-palmitoyl-sn-glycero-3-phosphocholine, 1-stearoyl-sn -Glycero-3-phosphocholine, bilirubin, choline, D-2-pipercolic acid, DL-indole-3-lactic acid, glycylglutamate, glycerophosphorylcholine, isomaltose, L - Arginine, L-glutamic acid, L-phenylalanine, L-tryptophan, NG,NG-dimethyl-L-arginine, tyramine, phenylacetic acid, (S)-lime Acid, 1-oleoyl-L-alpha-lysophosphatidic acid, 1-palmitoyl lysophosphatidic acid, alpha-mangostin, arachidonic acid (peroxide free), citramalic acid, cyclamate /salt, D-galactate/salt, D-threitol, D(-)-beta-hydroxybutyric acid, inositol galactoside, glyceric acid, L-aspartic acid, L-carnosine, L - In the group consisting of malic acid, N-acetyl-L-aspartic acid, N-acetylneuraminic acid, norethindrone acetate, phosphorylcholine, sn-glycero-3-phosphoethanolamine and succinate/salt;

(3) Protein, wherein the protein is at least one of the proteins selected from the group consisting of COLEC10, CPQ, LDHB, ACAN, KRT1, ARHGDIB, POSTN, LAMAS, CPNI, PROC, CIS, HPX, SUPT6H, BCAR3, BCHE, SOX30, C1R, PON1, CFHR4, PROZ, IGKV1D-16, APOC-3, COMP, SELENOP, PMFBP1, GSN, SELL, FUCA2, HLA-B, FGFBP2, FKBP1A, HSPE1, SAA1, PGD, CDH1, SOD1, ISLR, COROIA, ZYX, RLBP1, IDH1, IGKV6-21, SORL1, FAM3C, CST6, COTL1, PIGR, A0A1W6IYJ2, PRDX1, CAT, PROS1, CD5L, HMFT1766, C1QA, KRT2, and APP; and

(4)Lipid subclass substances or lipid molecules, wherein the lipid subclass substance is at least one of the lipid subclass substances selected from the group consisting of acetylcarnitine, ceramide, monosaccharide ceramide, disaccharide ceramide, glycosyl ceramide series, trisaccharide Ceramide, cholesteryl ester, cardiolipin, ganglioside, lysophosphatidylcholine, lysophosphatidylethanolamine, monogalactosyldiacylglyceride, phosphatidic acid, phosphatidylcholine, phosphatidylethanolamine, phytosphingosine, phosphatidylinositol, phosphatidylserine, sphingomyelin and triglyceride, and wherein the lipid molecule is at least one of the lipid molecules selected from the group consisting of PS(39:4)-H, SM(d45:6)+H, PC(14:0/20:4)+HCOO, SM(d20:0/18:1)+HCOO, PS(49:4)-H, LPC(26:0)+HCOO, SM(d20:0/16: 0)+HCOO, PA(50:4)-H, LPC(20:4)+HCOO, PC(36:5)+H, LPC(20:1)+HCOO, PC(38:8)+H, PC(46:5 )+H, PE(18:0p/22:5)-H, SM(d22:0/16:0)+HCOO, PC(15:0/20:4)+HCOO, SM(d18:0/20:4)+HCOO, LPC(22:4)+HCOO, PI(18:0/22:4)-H, PC(16:1/18:2)+HCOO, PS(40:4)-H, Cer(d20:0/ 24:1)+H, SM(d37:0)+HCOO, PC(17:1/16:0)+HCOO, SM(d20:0/22:5)+HCOO, PC(44:11)+H, SM(d32:0)+HCOO, LPC(22:1)+H, PC(33:0e)+H, SM(d56:2+pO)+H, SM(d42:1)+HCOO, LPC(17:1)+HCOO, SM(d17:0/18:2)+HCOO, Cer(d18:1/24:0)+H, SM(d36:3)+HCOO, AcCa(14:2)+H, Cer(d18:1/24:0 O)+H, PE(20:0p/20:4)-H, LPC(26:1)+HCOO, PC(37:5)+H, SM(d44:1)+HCOO, Cer(d18:1/26:1)+H, PE(40 :6e)+H, TG(18:1/20:2/22:4)+NH4, SM(d34:3)+H, PE(40:4)-H, SM(d22:0/18:2)+HCOO, Cer(d18:0/22:0)+H, SM(d44:0)+HCOO, PI(16:1/20:4)-H, PC(20:3/20:4)+HCOO, PE(38:1p )-H and SM(d39:0)+HCOO.

2. The use according to claim 1, wherein the fatty acids are at least 2 of the fatty acids defined in (1); the metabolites are at least 2 of the metabolites defined in (2); the protein are at least 2 of proteins defined in (3); or the lipid subclass substances or lipid molecules are at least 2 of the lipid subclass substances or lipid molecules defined in (4).

3. The use according to claim 1, wherein the fatty acids are at least 3 of the fatty acids defined in (1); the metabolites are at least 3 of the metabolites defined in (2); the protein are at least 3 of proteins defined in (3); or the lipid subclass substances or lipid molecules are at least 3 of the lipid subclass substances or lipid molecules defined in (4).

4. The use according to claim 1, wherein the fatty acids are 1-6 of the fatty acids defined in (1); the metabolites are 1-6 of the metabolites defined in (2); the protein are 1-6 of proteins defined in (3); or the lipid subclass substances or lipid molecules are 1-6 of the lipid subclass substances or lipid molecules defined in (4).

5. The use according to any one of claims 1-4, wherein the fatty acid(s) in (1) is(are) selected from the group consisting of C8:0, C10:0, C11:0, C16:0, C20:0, C20:5N3, C22:1N9, C23:0 and C24:0; the metabolite(s) in (2) is(are) selected from the group consisting of choline, 1-aminocyclopropanecarboxylic acid, 1-myristoyl-sn-glycerol -3-Phosphocholine, D-2-pipericolic acid, DL-indole-3-lactic acid, glycyl glutamic acid, L-glutamic acid, L-phenylalanine, L-tryptophan, tyramine, glyceric acid, cyclamate, D-galactate/salt and sn-glycero-3-phosphoethanolamine; the protein(s) in (3) is(are) selected from the group consisting of HSPE1, PGD , CDH1, ISLR, CPQ, IDH1, PIGR, POSTN, PROC, PROZ, PMFBP1, SELL, A0A1W6IYJ2, CAT, PROS1, CD5L, C1QA, KRT1 and FGFBP2; or the lipid subclass substance(s) in (4) is(are) selected from the group consisting of Cer, ChE, LPC, LPE, PA, PC, PE, PI, PS and SM, the lipid molecule(s) in (4) is(are) selected from the group consisting of PS(39:4)-H, SM(d45:6)+H, PC(14:0/20:4)+HCOO, SM(d20:0/18:1)+HCOO, PS(49:4)-H, LPC(26:0)+HCOO, SM(d20:0/16 :0)+HCOO, PA(50:4)-H and LPC(20:4)+HCOO.

6. The use according to claim 5, wherein the fatty acid(s) in (1) is(are) selected from the group consisting of C20:5N3, C22:1N9, C23:0 and C24:0;

    the metabolite(s) in (2) is(are) selected from the group consisting of 1-myristoyl-sn-glycero-3-phosphocholine, glycyl glutamic acid, L-phenylalanine, DL-indole-3-lactic acid, L-tryptophan and glyceric acid;
    the protein(s) in (3) is(are) selected from the group consisting of HSPE1, CDH1, ISLR, IDH1, COTL1 and FGFBP2; or
    the lipid subclass substance(s) in (4) is(are) selected from the group consisting of LPC, LPE, PA, PC and PE or the lipid molecule(s) in (4) is(are) selected from the group consisting of PS(39:4)-H, SM(d45:6)+H, PC(14:0/20:4)+HCOO and SM(d20:0/18:1)+HCOO.

7. The use according to any of the claims 1-4, wherein the fatty acid(s) in (1) is(are) selected from the group consisting of C10:0, C14:0, C16:0, C18:2N6, C20:0, C20: IN9, C20:5N3, C22:1N9, C24:0 and C8:0.

8. The use according to Claim 7, the fatty acid(s) in (1) is(are) selected from the group consisting of C10:0, C16:0, C20:0, C20:5N3 and C22:1N9.

9. A test kit comprising the composition described in any of Claims 1-8 and instructions for using the kit.

10. The test kit according to Claim 9, wherein the samples tested by the kit are selected from the group consisting of the subject's whole blood, plasma, serum, lymphocyte suspension, cerebrospinal fluid, bone marrow, pleural effusion, urine, saliva, and abdominal effusion.

11. The test kit according to Claim 9, the samples tested by the kit are selected from the group consisting of umbilical cord blood and peripheral blood.

12. The test kit according to Claim 9, wherein, said kit is a dry blood spot kit.

13. A drug for treating hemangioma in infants and young children comprises a substance that regulates the expression of selected LDHB, KRT1, BCHE, CFHR4, CPQ, APOC-3, ARHGDIB, and LAMA5.

Figure 1

Figure 2

Figure 3

Figure 4

Figure 5

Figure 6

Figure 7

Figure 8

Figure 9

Figure10

Figure11

Figure12

Figure13

Figure 14

ROC curve

AUC=0.934

P<0.0001

Figure 15

ROC curve

AUC=0.952

P<0.0001

Figure 16

ROC 曲线

AUC=0.960

P<0.0001

Figure 17

ROC curve

AUC=0.966

P<0.0001

Figure 18

AUC:0.955 (95%CIs:0.904-1.000)

Figure 19

AUC:0.951 (95%CIs:0.904-0.998)

Figure 20

## INTERNATIONAL SEARCH REPORT

International application No.

**PCT/CN2022/079542**

### A. CLASSIFICATION OF SUBJECT MATTER

G01N 33/574(2006.01)i;  G01N 33/92(2006.01)i;  G01N 30/02(2006.01)i

According to International Patent Classification (IPC) or to both national classification and IPC

### B. FIELDS SEARCHED

Minimum documentation searched (classification system followed by classification symbols)

G01N

Documentation searched other than minimum documentation to the extent that such documents are included in the fields searched

Electronic data base consulted during the international search (name of data base and, where practicable, search terms used)

ENTXTC; CNTXT; VEN; ENTXT; MEDNPL; CJFD; WFNPL; Web of Science: 婴幼儿血管瘤, 脂肪酸, 脂质组, infantile hemangioma, fatty acids, lipidomics

### C. DOCUMENTS CONSIDERED TO BE RELEVANT

| Category* | Citation of document, with indication, where appropriate, of the relevant passages | Relevant to claim No. |
|---|---|---|
| PX | JIANG, Chenghong et al. "Metabolic Profiling Revealed Prediction Biomarkers for Infantile Hemangioma in Umbilical Cord Blood Sera: A Prospectiv Study." *J.Proteome Res.*, Vol. 21, 28 July 2021 (2021-07-28), entire document | 1-12 |
| X | CN 109298115 A (HUMAN METABOLOMICS LNSTITUTE LNC.) 01 February 2019 (2019-02-01) description, paragraphs 7 and 27 | 9-12 |
| Y | CN 109298115 A (HUMAN METABOLOMICS LNSTITUTE LNC.) 01 February 2019 (2019-02-01) description, paragraphs 7 and 27 | 1-8 |
| X | CN 111650286 A (SHANGHAI APT BIOTECHNOLOGY CO., LTD.) 11 September 2020 (2020-09-11) claim 4 | 9-12 |
| Y | CN 111650286 A (SHANGHAI APT BIOTECHNOLOGY CO., LTD.) 11 September 2020 (2020-09-11) claim 4 | 1-8 |

✓ Further documents are listed in the continuation of Box C.  ✓ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **24 May 2022** | **22 June 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

<table>
<tr><td colspan="2" rowspan="2"><b>INTERNATIONAL SEARCH REPORT</b></td><td>International application No.</td></tr>
<tr><td><b>PCT/CN2022/079542</b></td></tr>
</table>

**C.     DOCUMENTS CONSIDERED TO BE RELEVANT**

| Category* | Citation of document, with indication, where appropriate, of the relevant passages | Relevant to claim No. |
|---|---|---|
| X | CN 103616465 A (ZHEJIANG UNIVERSITY) 05 March 2014 (2014-03-05)<br>    description, paragraphs 66 and 137-139 | 9-12 |
| Y | CN 103616465 A (ZHEJIANG UNIVERSITY) 05 March 2014 (2014-03-05)<br>    description, paragraphs 66 and 137-139 | 1-8 |
| X | US 2019113530 A1 (CBMED GMBH CENTER FOR BIOMARKER RES IN MEDICINE et al.) 18 April 2019 (2019-04-18)<br>    description, paragraphs 33-39 and 46 | 9-12 |
| Y | YANG, Kaiying et al. "Microarray Expression Profile of mRNAs and Long Noncoding RNAs and the Potential Role of PFK-1 in Infantile Hemangioma."<br>Vol. 16, No. 1, 11 January 2021 (2021-01-11),<br>    abstract, p. 4, right column to p. 5, tables 3 and 4 | 1-8 |
| Y | CN 104619864 A (EXPANSCIENCE LAB) 13 May 2015 (2015-05-13)<br>    claim 28, and description, paragraph 208 | 1-8 |
| A | CN 102344956 A (SHANGHAI NINTH PEOPLE'S HOSPITAL, SHANGHAI JIAOTONG UNIVERSITY SCHOOL OF MEDICINE) 08 February 2012 (2012-02-08)<br>    entire document | 1-12 |
| A | WANG, Qiguang et al. "Combined Transcriptomic and Lipidomic Analysis Reveals Aberrant Lipid Metabolism in Central Nervous System Hemangioblastomas."<br>*Sci.Rep.*, 14 January 2021 (2021-01-14),<br>    entire document | 1-12 |
| A | JIANG, Chenghong et al. "Angiogenin: A Potential Serum Marker of Infantile Hemangioma Revealed by cDNA Microarray Analysis."<br>*Plast Reconstr.Surg.*, Vol. 134, No. 2, 31 August 2014 (2014-08-31),<br>    entire document | 1-12 |

Form PCT/ISA/210 (second sheet) (January 2015)

**INTERNATIONAL SEARCH REPORT**

International application No.

**PCT/CN2022/079542**

| Box No. III | Observations where unity of invention is lacking (Continuation of item 3 of first sheet) |
|---|---|

This International Searching Authority found multiple inventions in this international application, as follows:

[1] 1. Claims 1-8 (in part) and claims 9-12 (in part):

[2] an application of a composition in the preparation of a drug for diagnosing infantile hemangioma and/or monitoring progression and prognosis thereof, the composition comprising at least one of total fatty acids or total saturated fatty acids or total monounsaturated fatty acids or specific fatty acids; and a test kit comprising the fatty acid composition and a kit description.

[3] 2. Claims 1-8 (in part) and claims 9-12 (in part):

[4] an application of a composition in the preparation of a drug for diagnosing infantile hemangioma and/or monitoring progression and prognosis thereof, the composition comprising at least one of specific metabolites; and a test kit comprising the metabolite composition and a kit description.

[5] 3. Claims 1-8 (in part) and claims 9-12 (in part):

[6] an application of a composition in the preparation of a drug for diagnosing infantile hemangioma and/or monitoring progression and prognosis thereof, the composition comprising at least one of specific proteins; and a test kit comprising the protein composition and a kit description.

[7] 4. Claims 1-8 (in part) and claims 9-12 (in part):

[8] an application of a composition in the preparation of a drug for diagnosing infantile hemangioma and/or monitoring progression and prognosis thereof, the composition comprising at least one of specific lipid subspecies or lipid molecules; and a test kit comprising the lipid subspecies or lipid molecule composition and a kit description.

[9] 5. Claim 13: a drug for treating infantile hemangioma.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-12 (in part)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/CN2022/079542**

| Patent document cited in search report | | | Publication date (day/month/year) | Patent family member(s) | | | Publication date (day/month/year) |
|---|---|---|---|---|---|---|---|
| CN | 109298115 | A | 01 February 2019 | WO | 2020078478 | A1 | 23 April 2020 |
| | | | | EP | 3869192 | A1 | 25 August 2021 |
| | | | | US | 2022026398 | A1 | 27 January 2022 |
| CN | 111650286 | A | 11 September 2020 | None | | | |
| CN | 103616465 | A | 05 March 2014 | None | | | |
| US | 2019113530 | A1 | 18 April 2019 | CA | 3017044 | A1 | 14 September 2017 |
| | | | | JP | 2021177188 | A | 11 November 2021 |
| | | | | JP | 2019514020 | A | 30 May 2019 |
| | | | | AU | 2017230870 | A1 | 04 October 2018 |
| | | | | EP | 3427064 | A1 | 16 January 2019 |
| | | | | KR | 20190019902 | A | 27 February 2019 |
| | | | | WO | 2017153472 | A1 | 14 September 2017 |
| CN | 104619864 | A | 13 May 2015 | FR | 2993282 | A1 | 17 January 2014 |
| | | | | CA | 2878971 | A1 | 16 January 2014 |
| | | | | US | 2015285787 | A1 | 08 October 2015 |
| | | | | JP | 2015528699 | A | 01 October 2015 |
| | | | | EP | 2872647 | A1 | 20 May 2015 |
| | | | | ES | 2838548 | T3 | 02 July 2021 |
| | | | | MX | 2015000583 | A | 14 August 2015 |
| | | | | KR | 20150028353 | A | 13 March 2015 |
| | | | | WO | 2014009566 | A1 | 16 January 2014 |
| CN | 102344956 | A | 08 February 2012 | None | | | |

Form PCT/ISA/210 (patent family annex) (January 2015)